# EUROPEAN PATENT APPLICATION

(11) **EP 2 727 937 A1**
(43) Date of publication of application: **07.05.2014**
(21) Application number: 12803735.5
(22) Date of filing: 28.06.2012
(51) Int. Cl.: C07K 14/71, A61K 38/00, A61K 39/395, A61P 1/04, A61P 3/10, A61P 9/10, A61P 11/00, A61P 13/12, A61P 17/00, A61P 19/02, A61P 25/00, A61P 29/00, A61P 31/18, A61P 35/00, A61P 35/04, A61P 37/00, A61P 37/06, C07K 16/28

(54) **SOLUBLE INTEGRIN 4 MUTANT**

(30) Priority: 30.06.2011 JP 2011146164
(71) Applicant: Immuno-Biological Laboratories Co., Ltd., Fujioka-shi Gunma 375-0005 (JP); Gene Techno Science Co., Ltd., Chuo-ku Sapporo-shi Hokkaido 060-0002 (JP)
(72) Inventor: SEITO, Tsutomu, Fujioka-shi Gunma 375-0005 (JP); KON, Shigeyuki, Sapporo-shi Hokkaido 001-0017 (JP)
(74) Representative: Woods, Geoffrey Corlett
(86) International application number: PCT/JP2012/004191
(87) International publication number: WO 2013/001819

(57) **Abstract**

The present invention addresses the problem of providing a novel substance capable of interfering with various functions of integrin α4, and/or providing a novel substance capable of interfering with both integrin α4 and integrin α9. The present invention provides an integrin α4 mutant peptide having one portion of the extracellular domain of human integrin α4, and the like, and in concrete terms relates to a peptide and the like having the amino acid sequence of Sequence No. 4 through 9, and a pharmaceutical composition comprising as the active ingredient the same peptide.

## Description

### [Technical Field]

The present invention relates to the field of treatment or prevention of diseases in which integrin α4 and integrin α9 are involved. More specifically, the present invention relates to a method for treatment or prevention of diseases in which integrin α4 and integrin α9 are involved which uses a soluble integrin α4 mutant.

### [Background Art]

Integrin (hereinafter referred to as the "ITG") is a transmembrane receptor protein in which an α-chain and a β-chain form a heterodimer at 1:1. It is known that integrin α4 (hereinafter referred to as the "ITG-α4") is principally expressed on lymphocytes and tumor cells, and binds to ligands such as vascular cell adhesion molecule-1 (VCAM1), fibronectin (Fn), junction adhesion molecule-2 (JAM2), mucosal vascular addressin cell adhesion molecule- 1 (MAdCAM1), human lymphocyte expression metalloproteinase-like disintegrin-like cysteine rich protein family member (MDC-L; disintegrin and metalloprotease 28 (ADAM28)), von Willebrand factor (pp-vWF) and osteopontin (OPN) (Non Patent Literatures 1 and 2). It is regarded that ITG-α4 binds to such a ligand so as to exhibit various functions such as cell adhesion, migration (for example, migration of lymphocytes, monocytes or eosinocytes to an inflammation site), and homing of lymphocytes. It has been found that ITG-α4 is involved in cancer metastasis (Non Patent Literature 3), multiple myeloma (Patent Literature 1), inflammatory diseases such as rheumatoid arthritis, bronchitis, inflammatory bowel disease, Crohn disease and multiple sclerosis (Non Patent Literature 4), acute central nervous system damage (Patent Literature 2), and HIV (Patent Literature 3). It has been reported that an anti-ITG-α4 antibody shows a certain effect in models of T cell dependent autoimmune diseases such as experimental allergic encephalomyelitis, hypersensitivity and type I diabetes. Furthermore, it is also known that an anti-ITG-α4 antibody is effective for suppressing allergic pneumonia, immunocomplex-mediated pneumonopathy, acute nephrotoxic nephritis, and sclerema and deposition of fibrin in delayed hypersensitivity, and suppresses rejection occurring in vascularized heart allograft (Non Patent Literature 1).

Since ITG-α4 is thus involved in the diseases, it is expected to use an inhibitor of ITG-α4 for a medicine. Actually, substances to inhibit the functions of ITG-α4, such as an anti-ITG-α4 antibody, an ITG-α4 antagonist (Patent Literature 4 and the like), and an ITG-α4 receptor antagonist (Patent Literature 5 and the like) have been examined, and an anti-ITG-α4 antibody Tysabri has already been approved as a therapeutic agent for multiple sclerosis and Crohn disease. Furthermore, a treatment method targeting a functional motif in an intracytoplasmic domain of ITG-α4 (Patent Literature 6) has been reported.

In using an antibody is used as a pharmaceutical, its effect varies according to its epitope. It is known that α4β1ITG strongly binds to its ligand on a sequence (LDV sequence (SEQ ID NO: 2) or QIDSPL sequence (SEQ ID NO: 3) which is different from a RGD sequence (SEQ ID NO: 1) to which ITG-α5β1 binds (Non Patent Literatures 1 and 2). It was known that each binding of ITG-α4 to its ligand has different characteristics depending on the ligand. For example, although a binding sites of ITG-α4 to Fn and VCAM1 mutually overlap, binding of ITG-α4 to VCAM1 requires a help of a calcium ion whereas binding to Fn does not require such ion, and some antibodies only inhibit binding of ITG-α4 to Fn. Moreover, it was known that three kinds of epitopes not overlapping one another exist as principal epitopes of anti-ITG-α4 antibodies (Non Patent Literature 1).

On the other hand, integrin α9 (hereinafter referred to as "ITG-α9") is known as an ITG having common ligands with ITG-α4. It has been reported that ITG-α9 is involved in exacerbation of experimental autoimmune encephalomyelitis (EAE), a mouse model of multiple sclerosis (Non Patent Literature 5). The present inventors produced an antibody against ITG-α9 and found a treatment effect for rheumatoid arthritis (Non Patent Literature 6 and Patent Literature 7).

Since ITG-α9 has common ligands with ITG-α4, ITG-α9 is regarded to have functions similar to those of ITG-α4. Furthermore, an amino acid sequence of ITG-α9 has 39% homology to that of ITG-α4, which are known as the highest homology among ITGs (Non Patent Literature 7).

The present inventors have discovered that an α9 mutant (SFα9), an alternative splicing variant of ITG-α9 consisting a part of an extracellular region of ITG-α9 and a novel C-terminal amino acid, is present in vivo, and reported the result of analysis of the structure and functions of the SFα9 (Non Patent Literature 8). On the other hand, relating to ITG-α4 mutant, only polymorph of ITG-α4 was reported (Patent Literature 8), however no report has been made on an alternative splicing variant.

### [Citation List]

### Patent Literatures

Patent Literature 1: International Publication No. WO00/15247
Patent Literature 2: International Publication No. WO01/43774
Patent Literature 3: International Publication No. WO2008/140602
Patent Literature 4: International Publication No. 2010/126914
Patent Literature 5: International Publication No. 2006/096807
Patent Literature 6: International Publication No. WO2006/112738
Patent Literature 7: International Publication No. WO2008/007804
Patent Literature 8: International Publication No. WO00/17394

### Non Patent Literatures

Non Patent Literature 1: Roy R. Lobb., et al. (1994) J. Clin. Invest., 94: 1722-1728
Non Patent Literature 2: Roberto Gonzalez-Amaro, et al. (2005) Immunology, 116: 289-296
Non Patent Literature 3: Holzmann, B., et al. (1998) Curr. Top. Microbiol. Immunol., 231: 125-141
Non Patent Literature 4: Jackson, D. Y. (2002) Curr. Pharm. Des., 8: 1229-1253
Non Patent Literature 5: Kanayama, M., et al. (2009) J. Immunol., 182: 8015-8025
Non Patent Literature 6: Palmer, E. L., et al. (1993) J. Cell. Biol., 123: 1289-1297
Non Patent Literature 7: International Immunology Meeting, PP-038-31
Non Patent Literature 8: Kon, S., et al. (2011) Exp. Cell. Res. 317: 1774-84

### [Summary of Invention]

Since ITG-α4 and ITG-α9 have common ligands and are regarded to be involved in the onset of similar diseases such as EAE, a substance capable of simultaneously inhibiting ITG-α4 and ITG-α9 is expected to be an effective therapeutic agent for the diseases relating to ITG-α4 and ITG-α9. Furthermore, it is known that cancer cells of melanoma and the like express both ITG-α4 and ITG-α9 (J Cell Physiol. (2007) 212: 368-74; Exp Cell Res.

(2009) 315: 3312-24). A cancer metastasis is known to involve steps of rolling in blood vessels, adhesion, and transmigration out of the blood vessels. ITG is regarded to play a significant function in the adhesion. When a common ligand of ITG-α4 and ITG-α9 is expressed on vascular endothelial cells, it is regarded that inhibition of binding between one of the integrins and said ligand by using an antibody or the like cannot block metastasis, and it is necessary to use an antibody against both of ITG-α4 and ITG-α9 in order to block such binding. Actually, it has been reported that the von Willebrand factor (vWF), a common ligand of ITG-α4 and ITG-α9, is produced from vascular endothelial cells (J Clin Invest. 1978 Jun, 61(6): 1498-507). It is reported that both of ITG-α4 and ITG-α9 are expressed in lymphatic vessels (Nat Rev Cancer. (2008) 8: 604-17). Accordingly, a substance capable of simultaneously inhibiting ITG-α4 and ITG-α9 is regarded to be an effective antimetastatic agent. Furthermore, influence of ITG-α4 inhibition and ITG-α9 inhibition on lymphangiogenesis and the function of lymphatic vessels has been reported (Cancer Res. (2010) 70: 3042-51, EMBO J. (2005) 24: 2885-95), and it is expected that a novel efficient lymphangiogenesis inhibitor can be provided by simultaneously inhibiting ITG-α4 and ITG-α9.

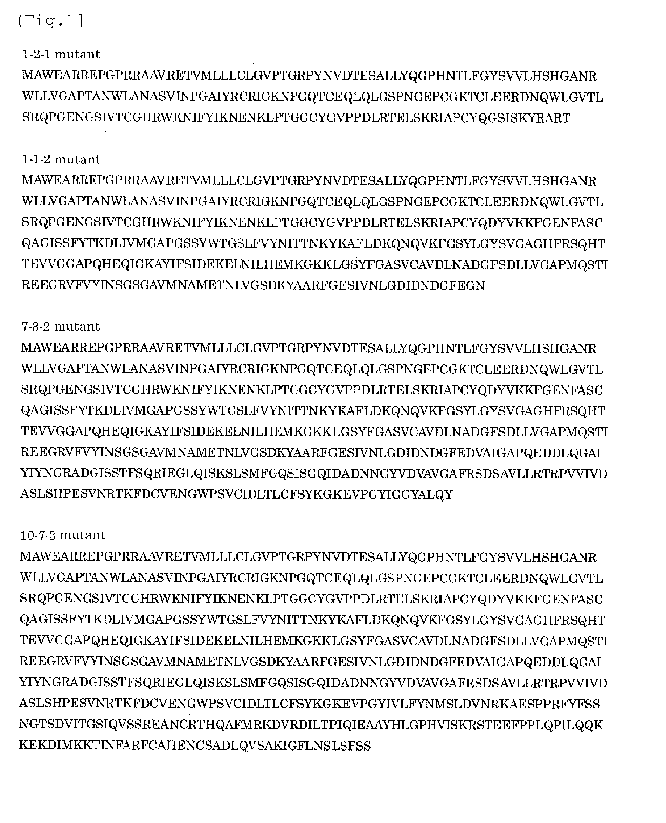

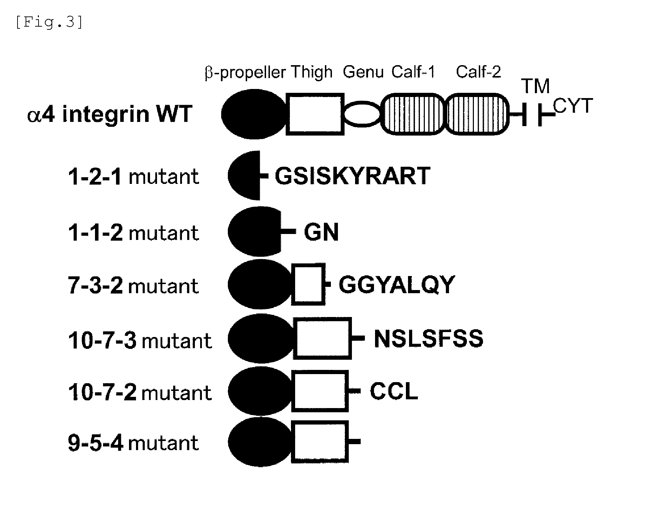

The present inventors tried to search for an effective inhibitor for ITG-α4 and identified novel alternative splicing variants of ITG-α4. As a result, the present inventors have succeeded in identifying six novel alternative splicing variants (Figures 1 to 3 and SEQ ID NOs: 4 to 9). Surprisingly, analysis of the structures of the obtained alternative splicing variants of ITG-α4 revealed that five of six obtained alternative splicing variants unexpectedly have novel and peculiar amino acid sequences at the C-terminal which are derived from introns but not derived from human ITG-α4 (SEQ ID NO: 10) (said novel amino acid sequence at the C-terminal of the each ITG-α4 alternative splicing variant is shown in Figure 3).

From analysis of the functions of the obtained ITG-α4 alternative splicing variants, the present inventors have found that these alternative splicing variants can inhibit bindings of ITG-α4 to a plurality of different ligands. Furthermore, the present inventors have found that the obtained ITG-α4 alternative splicing variants can inhibit bindings of ITG-α9 to its ligands in addition to bindings of ITG-α4 to its ligands.

An object of the present invention is to provide a novel substance capable of inhibiting various functions of ITG-α4 and/or to provide a novel substance capable of inhibiting both of ITG-α4 and ITG-α9. The present invention is on the basis of finding of novel alternative splicing variants of ITG-α4. Specifically, the present invention relates to the following inventions (1) to (17):
(1) An integrin α4 mutant peptide comprising a part of an extracellular region of human integrin α4 (SEQ ID NO: 10);
(2) The peptide of (1), wherein the extracellular region of human integrin α4 (SEQ ID NO: 10) includes an amino acid sequence derived from a β-propellor domain of human integrin α4 (SEQ ID NO: 10);
(3) The peptide of (1), wherein the extracellular region of human integrin α4 (SEQ ID NO: 10) consist of a sequence selected from SEQ ID NO: 9 and SEQ ID NOs: 11 to 14;
(4) The peptide of any one of (1) to (3), wherein a sequence selected from SEQ ID NOs: 15 to 19 is bonded to a C-terminal of the extracellular region of human integrin α4 (SEQ ID NO: 10);
(5) A peptide comprising a sequence selected from SEQ ID NOs: 4 to 9;
(6) The peptide of any one of (1) to (5), which binds to a ligand of integrin α4 and/or inhibits binding of integrin α4 to said ligand;
(7) The peptide of (6), wherein the ligand of integrin α4 is a substance selected from the group consisting of VCAM1, Fn, JAM2, MAdCAM1, MDC-L (ADAM28), pp-vWF and OPN;
(8) The peptide of (6), wherein the ligand of integrin α4 is pp-vWF or OPN;
(9) The peptide of (6), wherein the ligand of integrin α4 is a peptide comprising an amino acid sequence of SEQ ID NO: 20 or 21;
(10) The peptide of any one of (1) to (9), which further binds to a ligand of integrin α9 and/or inhibits binding of integrin α9 to said ligand;
(11) The peptide of (10), wherein the ligand of integrin α9 is a substance selected from the group consisting of VCAM1, Fn, JAM2, MAdCAM1, MDC-L (ADAM28), pp-vWF and OPN;
(12) The peptide of (10), wherein the ligand of integrin α9 is pp-vWF or OPN;
(13) The peptide of (10), wherein the ligand of integrin α9 is a peptide comprising an amino acid sequence of SEQ ID NO: 20 or 21;
(14) The peptide of any one of (1) to (13), which is a soluble peptide;
(15) A pharmaceutical composition containing the peptide of any one of (1) to (14) as an active ingredient;
(16) An antibody that specifically recognizes (or binds to) the peptide of any one of (1) to (14); and
(17) The antibody of (16) that does not recognize (or does not bind to) integrin α4.

In the present invention, "integrin α4 mutant" and "ITG-α4 mutant" mean the same, that is an alternative splicing variant peptide of ITG-α4 which are derived from the genome of ITG-α4 but produced through different splicing from ITG-α4, or a peptide having substantially the same amino acid sequence as said alternative splicing variant peptide of ITG-α4. The "integrin α4 mutant" herein includes a part of an extracellular region of human ITG-α4 (corresponding to a portion of the 1st to 977th amino acid sequence of SEQ ID NO: 10) but includes neither a transmembrane region nor an intracellular region. The presumed domains of ITG-α4 were reported as that obtained by homology analysis of the amino acid sequence of ITG-α4 comparing with that of integrin αv which was already analyzed for steric structure (Science (2001), 294. 339-345) (Proc Natl Acad Sci USA. (1997) 94: 65-72). Specifically, ITG-α4 extracellular region is known to have domains designated as: a signal sequence (corresponding to a portion of amino acid sequence positions from 1 to 33 of SEQ ID NO: 10); β-propellor (corresponding to a portion of amino acid sequence positions from 34 to 465 of SEQ ID NO: 10); Thigh (corresponding to a portion of amino acid sequence positions from 466 to 618 of SEQ ID NO: 10); Genu (corresponding to a portion of amino acid sequence positions from 619 to 626 of SEQ ID NO: 10); Calf1 (corresponding to a portion of amino acid sequence positions from 627 to 770 of SEQ ID NO: 10); and Calf2 (corresponding to a portion of amino acid sequence positions from 771 to 977 of SEQ ID NO: 10). As the part of the extracellular region of human ITG-α4 of the present invention, it preferably is the β-propellor domain and/or the Thigh domain. The part of the extracellular region of human ITG-α4 herein has, for example, an amino acid sequence derived from the β-propellor domain of human ITG-α4. Here, the amino acid sequence derived from the β-propellor domain may be a sequence of any site as long as it is a part of the amino acid sequence of the β-propellor domain positioned at the N-terminal of ITG-α4. The part of the extracellular region of human ITG-α4 may have, for example, 50 or more, 100 or more, 150 or more, 180 or more, or 185 or more amino acids, derived from the amino acid sequence of the β-propellor domain (consisting of 432 amino acids). Furthermore, the amino acid sequence derived from the β-propellor domain of human ITG-α4 preferably contains the amino acid at position 34 sequence of SEQ ID NO: 10. Moreover, the part of the extracellular region of human ITG-α4 having an amino acid sequence derived from the β-propellor domain of human ITG-α4 may consisting of merely an amino acid sequence derived from the β-propellor domain, or may have, in addition to an amino acid sequence derived from the β-propellor domain, an amino acid sequence derived from the extracellular region of human ITG-α4 other than the β-propellor domain. More specifically, the part of the extracellular region of human ITG-α4 herein may include any one of the amino acid sequence of SEQ ID NOs: 11 to 14 (or any one of the amino acid sequences which lack a signal sequence consisting of the positions from 1 to 33 of said amino acid sequence), or may include any one of amino acid sequences of the positions from 1 to 185, the positions from 1 to 384, the positions from 1 to 513, and the positions from 1 to 640 of SEQ ID NO: 10 (or any one of the amino acid sequences of the positions from 34 to 185, the positions from 34 to 384, the positions from 34 to 513, and the positions from 34 to 640 of SEQ ID NO: 10).

In the present application, the "integrin α4 mutant" may contain an amino acid sequence which is not derived from ITG-α4 (hereinafter referred to as the "ITG-α4 mutant specific amino acid sequence"). The "ITG-α4 mutant specific amino acid sequence" preferably is a sequence derived from intron of ITG-α4, and specifically can be an amino acid sequence represented by any one of SEQ ID NOs: 15 to 19.

In other words, in one aspect, an "integrin α4 mutant" herein is a peptide consisting of a part of the extracellular region of human ITG-α4 alone or a peptide consisting of a part of the extracellular region of human ITG-α4 and the ITG-α4 mutant specific amino acid sequence (hereinafter collectively designated as an "alternative splicing variant of ITG-α4"). Specifically, the ITG-α4 mutant herein may be a peptide comprising of an amino acid sequence of any one of SEQ ID NOs: 4 to 9 (designated as a "1-2-1 mutant" (SEQ ID NO: 4), a "1-1-2 mutant" (SEQ ID NO: 5), a "7-3-2 mutant" (SEQ ID NO: 6), a "10-7-3 mutant" (SEQ ID NO: 7), a "10-7-2 mutant" (SEQ ID NO: 8) and a "9-5-4 mutant" (SEQ ID NO: 9), respectively). Furthermore, the alternative splicing variant of ITG-α4 herein has an activity to bind to a ligand of ITG-α4 and/or to inhibit binding of ITG-α4 to its ligand, and/or has an activity to bind to a ligand of ITG-α9 and/or to inhibit binding of ITG-α9 to its ligand.

In another aspect, an "integrin α4 mutant comprising a part of an extracellular region of human integrin α4" herein includes a peptide which is substantially the same as the alternative splicing variant of ITG-α4. Here, the peptide which is substantially the same means a peptide which has highly homologous amino acid sequence to said alternative splicing variant of ITG-α4, a peptide which is encoded by a DNA capable of hybridizing with a DNA encoding said alternative splicing variant of ITG-α4 under stringent conditions, and/or a peptide in which a small number of amino acid residues are substituted, deleted, added and/or inserted. These peptides which are substantially the same as the alternative splicing variant of ITG-α4 has biological activity equivalent to that of the alternative splicing variant of ITG-α4 of the present specification.

Herein, highly homologous amino acid sequence means to have an amino acid sequence with homology of 80%, 85%, 90%, 95%, 98% or 99% to the alternative splicing variant of ITG-α4. For example, a peptide which has highly homologous amino acid sequence to the alternative splicing variant of ITG-α4 may be a peptide having an amino acid sequence with homology of 80%, 85%, 90%, 95%, 98% or 99% to any one of the amino acid sequences of SEQ ID NOs: 4 to 9. Here, the homology of an amino acid sequence can be determined by a method usually employed by those skilled in the art using a known program such as BLAST or FASTA.

Herein, a DNA capable of hybridizing with a DNA encoding the alternative splicing variant of ITG-α4 under stringent conditions means a DNA which hybridizes with the DNA encoding the alternative splicing variant of ITG-α4 under hybridization conditions usually employed by those skilled in the art. For example, a DNA wihch hybridizes with a DNA encoding the alternative splicing variant of ITG-α4 under stringent conditions may be a DNA which hybridizes with a DNA encoding any one of the amino acid sequences of SEQ ID NOs: 4 to 9 under stringent conditions. Whether the candidate DNA can hybridize under stringent conditions can be determined as whether it hybridizes by a method described in Molecular Cloning, A Laboratory Manual, Second Edition, Cold Spring Harbor Laboratory Press (1989). For example, the stringent hybridization conditions may be conditions in which the hybridization is performed in 6 x SSC (0.9 M NaCl, 0.09 M trisodium citrate) or 6 x SSPE (3M NaCl, 0.2 M NaH₂PO₄. 20 mM EDTA·2Na, pH 7.4) at 42°C, and the resultant is washed with 0.5 x SSC at 42°C.

Herein, the peptide in which a small number of amino acid residues are substituted with, deleted from, added to and/or inserted into the alternative splicing variant of ITG-α4 means a peptide in which some amino acids of the amino acid sequence constituting the alternative splicing variant of ITG-α4 are substituted by other amino acids and/or deleted, and/or a peptide in which another amino acid sequence is added to the C-terminal or the N-terminal of the amino acid sequence constituting the alternative splicing variant of ITG-α4, and/or a peptide in which another amino acid is inserted into the amino acid sequence constituting the alternative splicing variant of ITG-α4. The peptide in which a small number of amino acid residues are substituted with, deleted from, added to and/or inserted into the alternative splicing variant of ITG-α4 can be, for example, a peptide in which a small number of amino acid residues are substituted with, deleted from, added to and/or inserted into a peptide consisting of any one of the amino acid sequences of SEQ ID NOs: 4 to 9. The number of amino acids to be substituted, deleted, added and/or inserted is not especially limited as long as the biological activity of the alternative splicing variant of ITG-α4 is not affected, and can be, for example, the number can be 1 to 10, 1 to 5, 1 to 4, or 1 to 3.

As described above, a peptide which is substantially the same as the alternative splicing variant of ITG-α4 has the biological activity equivalent to that of the alternative splicing variant of ITG-α4. Here, the "biological activity equivalent to that of the alternative splicing variant of ITG-α4" means an activity to bind to a ligand of ITG-α4 and/or to inhibit binding of integrin α4 to its ligand. Here, a ligand of ITG-α4 is not especially limited as long as it is a protein or a peptide already known as a ligand of ITG-α4, and is preferably VCAM1, Fn (fibronectin EIIIA (Fn-EIIIA) in particular), JAM2, MAdCAM1, MDC-L (ADAM28), pp-vWF or OPN, more preferably Fn-EIIIA, pp-vWF or OPN, and even more preferably a peptide having an amino acid sequence of SEQ ID NO: 20 or 21. In the meaning of the binding to a ligand of ITG-α4 and/or the inhibition of binding of integrin α4 to its ligand, said ligands need not to be all types of ligands of ITG-α4, but for example, it can be binding to some of the aforementioned ligands and/or can be the inhibition of binding of integrin α4 to some of the aforementioned ligands. Preferably, the peptide of the present invention binds to two or more ligands, and/or inhibits binding of integrin α4 to said ligands, and preferably said two or more ligands include Fn-EIIIA, pp-vWF and OPN, and more preferably include a peptide having an amino acid sequence of SEQ ID NO: 20 and a peptide having an amino acid sequence of SEQ ID NO: 21.

Preferably, the "biological activity equivalent to that of the alternative splicing variant of ITG-α4" means an activity to bind to a ligand of ITG-α9 and/or to inhibit binding of ITG-α9 to its ligand in addition to the activity to bind to a ligand of ITG-α4 and/or to inhibit binding of ITG-α4 to its ligand. Here, a ligand of ITG-α9 is not especially limited as long as it is a protein or a peptide already known as a ligand of ITG-α9, and is preferably VCAM1, Fn (fibronectin EIIIA (Fn-EIIIA) in particular), JAM2, MDC-L (ADAM28), pp-vWF or OPN, more preferably Fn-EIIIA, pp-vWF or OPN, and even more preferably a peptide having an amino acid sequence of SEQ ID NO: 20 or 21. In the meaning of the binding to a ligand of ITG-α9 and/or inhibiting binding of integrin α9 and its ligand, said ligands need not to be all types of ligands of ITG-α9, but for example, it can be binding to some of the aforementioned ligands and/or can be inhibition of binding of integrin α9 to some of the aforementioned ligands. Preferably, the peptide of the present invention binds to two or more ligands, and/or inhibits binding of integrin α9 to said ligands, and preferably said two or more ligands include Fn-EIIIA, pp-vWF and OPN, and more preferably include a peptide having an amino acid sequence of SEQ ID NO: 20 and a peptide having an amino acid sequence of SEQ ID NO: 21.

Herein, the activity to inhibit binding of ITG-α4 and its ligand and the activity to inhibit binding of ITG-α9 to its ligand can be replaced with an activity to inhibit binding of ITG-α4 expressing cells to an ITG-α4 ligand (cell adhesion of ITG-α4 expressing cells to an ITG-α4 ligand) and an activity to inhibit binding of ITG-α9 expressing cells expressing to an ITG-α9 ligand (cell adhesion of ITG-α9 expressing cells to an ITG-α9 ligand).

As described above, the ITG-α4 mutant of the present invention has the activity to bind to a ligand of ITG-α4 and/or to inhibit binding of ITG-α4 to its ligand, and/or the activity to bind to a ligand of ITG-α9 and/or to inhibit binding of ITG-α9 to its ligand. Preferably, the ITG-α4 mutant of the present invention has an activity to bind to a ligand of ITG-α4 and/or to inhibit binding of ITG-α4 to its ligand as well as an activity to bind to a ligand of ITG-α9 and/or to inhibit binding of ITG-α9 to its ligand. More preferably, the ITG-α4 mutant of the present invention binds to a common ligand for ITG-α4 and ITG-α9, or inhibits bindings of ITG-α4 and ITG-α9 to the common ligand for ITG-α4 and ITG-α9. Examples of the common ligand for ITG-α4 and ITG-α9 include Fn-EIIIA, pp-vWF and OPN.

Even more preferably, the "biological activity equivalent to that of the alternative splicing variant of ITG-α4" may include, in addition to the aforementioned activities, to be soluble. It can be determined whether a peptide of interest is soluble, for example, as follows: recombining a DNA encoding the peptide modified with FLAG or the like with host cells, culturing the resulting cells, and subsequently confirming the presence of the peptide in a culture supernatant by using an anti-FLAG antibody or the like. If the peptide has been released into the culture supernatant, it can be determined that the peptide is soluble. Herein, to be "soluble" means that most (more than half) of produced peptides are released into a culture medium and do not remain in cells or on cell surfaces, and it is not required that the produced peptides are entirely released into a culture medium.

Herein, the "biological activity equivalent to that of the alternative splicing variant of ITG-α4" basically means a qualitative property and the amplitude of the activity is not required as long as there is the aforementioned activity, but the amplitude is preferably equivalent (to have, for example, an active value within a range of ±25%, ±20%, ±15% or ±10%) to that of the alternative splicing variant of ITG-α4 (preferably, those of the peptides containing the amino acid sequences represented by SEQ ID NOs: 4 to 9).

Furthermore, an amino acid contained in the "integrin α4 mutant" herein may be appropriately chemically modified as appropriate. Moreover, the "integrin α4 mutant" of the present application may be a peptide that do not bind to the RGD sequence (SEQ ID NO: 1) and/or a peptide that do not inhibit binding of ITG (particularly, ITG-α4 and/or ITG-α9) to the RDG sequence (SEQ ID NO: 1).

In still another aspect, the present invention relates to an antibody which specifically recognizes the ITG-α4 mutant. The antibody of the present invention may be a polyclonal antibody or a monoclonal antibody, and is preferably a monoclonal antibody. In the present invention, a "monoclonal antibody" is one highly specific to an antigen and recognizing a single antigen. Furthermore, the antibody of the present invention includes a non-human animal antibody, an antibody having both of an amino acid sequence of a non-human animal antibody and an amino acid sequence of a human-derived antibody, and a human antibody. Examples of the non-human animal antibody include antibodies of a mouse, a rat, a hamster, a guinea pig, a rabbit, a dog, a monkey, a sheep, a goat, a chicken and a duck, and it is preferably an antibody of an animal from which a hybridoma can be prepared, and more preferably a mouse antibody. Examples of the antibody having both of an amino acid sequence of a non-human animal antibody and an amino acid sequence of a human-derived antibody include a human-type chimeric antibody and a humanized antibody. In the above, a "chimeric antibody" refers to an antibody in which a constant region of a non-human animal-derived antibody that specifically binds to an ITG-α4 mutant is altered by genetic engineering technique to have the same constant region of a human antibody, and is preferably a human-mouse chimeric antibody (see EP Patent Publication No. EP 0125023). A "humanized antibody" refers to an antibody in which a primary structure of a non-human animal-derived antibody that specifically binds to an ITG-α4 mutant other than the complementarity determining region (CDR) of an H chain and an L chain is altered, by genetic engineering technique, with a corresponding primary structure of a human antibody. Here, a CDR may be defined by either of Kabat et al. ("Sequences of Proteins of Immunological Interest", Kabat, E. et al., U.S. Department of Health and Human Services, 1983) or Chothia et al. (Chothia & Lesk (1987) J. Mol. Biol., 196: 901-917). A "human antibody" refers to a human antibody as an expression product of an antibody gene that is completely derived from human, and an example includes a monoclonal antibody produced by using a transgenic animal in which a gene relating to human antibody production has been introduced (see EP Patent Publication No. EP 0546073).

### [Advantageous Effects of Invention]

Since the ITG-α4 mutant of the present invention can inhibit various functions of ITG-α4, the ITG-α4 mutant can be used for treatment or prevention of various diseases which onset or exacerbation involve ITG-α4. In particular, since the ITG-α4 mutant of the present invention can inhibit not only binding of ITG-α4 to its ligand but also binding of ITG-α9 to its ligand, the ITG-α4 mutant can be used for treatment or prevention of various diseases which onset or exacerbation involve ITG-α4 and ITG-α9.

### [Brief Description of Drawings]

Figure 1 shows amino acid sequences of alternative splicing variants of ITG-α4, a 1-2-1 mutant, a 1-1-2 mutant, a 7-3-2 mutant and a 10-7-3 mutant.
Figure 2 shows amino acid sequences of alternative splicing variants of ITG-α4, a 10-7-2 mutant and a 9-5-4 mutant.
Figure 3 is a schematic diagram illustrating sequences of the alternative splicing variants of ITG-α4.
Figure 4 is a diagram illustrating genomic structures on chromosome 2 of the alternative splicing variants of ITG-α4.
Figure 5 is a picture of Western blotting as a result of confirming expression of each mutant in a culture supernatant of COS cells in which each of the six alternative splicing variants of ITG-α4 (the 1-1-2 mutant, the 1-2-1 mutant, the 7-3-2 mutant, the 10-7-3 mutant, the 10-7-2 mutant and the 9-5-4 mutant) was forcibly expressed.

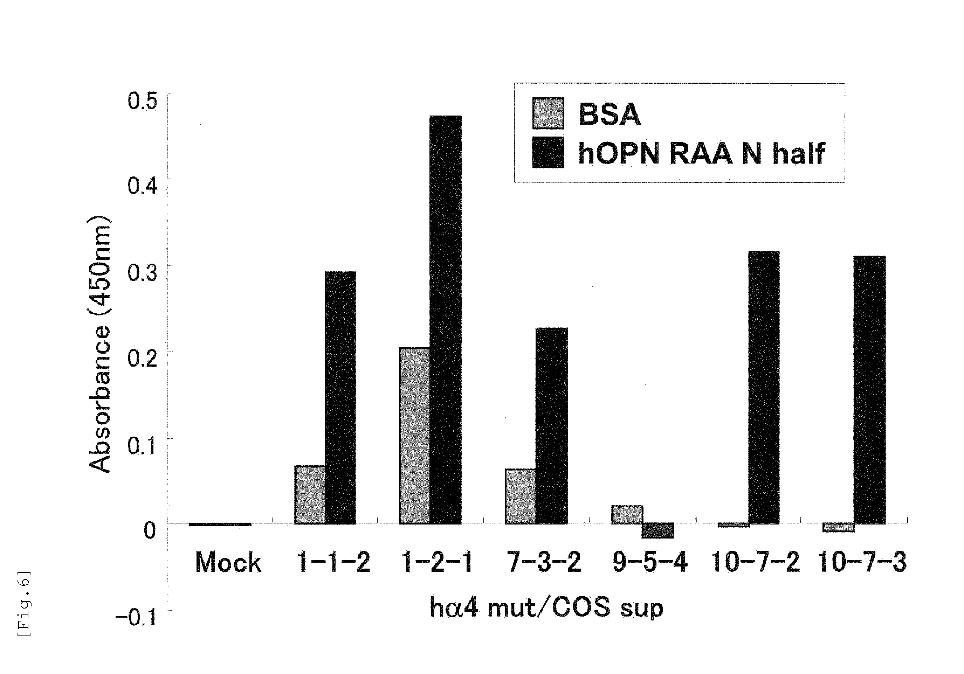

Figure 6 shows a graph of a result of ligand solid phase ELISA, which was conducted to investigate binding of each alternative splicing variant of ITG-α4 in a culture supernatant to human OPN RAA Nhalf protein that is a ligand of integrin α4 (Kon, S., et al., (2002) J. Cell. Biochem., 84: 420-432) or to a negative control (bovine serum albumin: BSA). The vertical axis indicates absorbance at 450 nm of a labeled antibody to be an index of the amount of the integrin α4 mutant bound to the ligand, and the horizontal axis indicates each alternative splicing variant of ITG-α4 used. Black bars indicate the bound of the alternative splicing variants of ITG-α4 to the human OPN RAA Nhalf protein, and gray bars indicate the bound of the alternative splicing variants of ITG-α4 to BSA.
Figure 7 shows a picture of Western blotting as a result of analyzing expression of endogenous 1-2-1 mutant. Each numerical value on the left of the picture indicates the molecular weight (kDa) of the protein. On above the photograph, "1-2-1/COS posi-con" indicates a culture supernatant of COS1 cells in which the 1-2-1 mutant was forcibly expressed, "Jurkat sup" indicates a culture supernatant of Jurkat cells, and "Rec-1 sup" indicates a culture supernatant of Rec-1 cells.

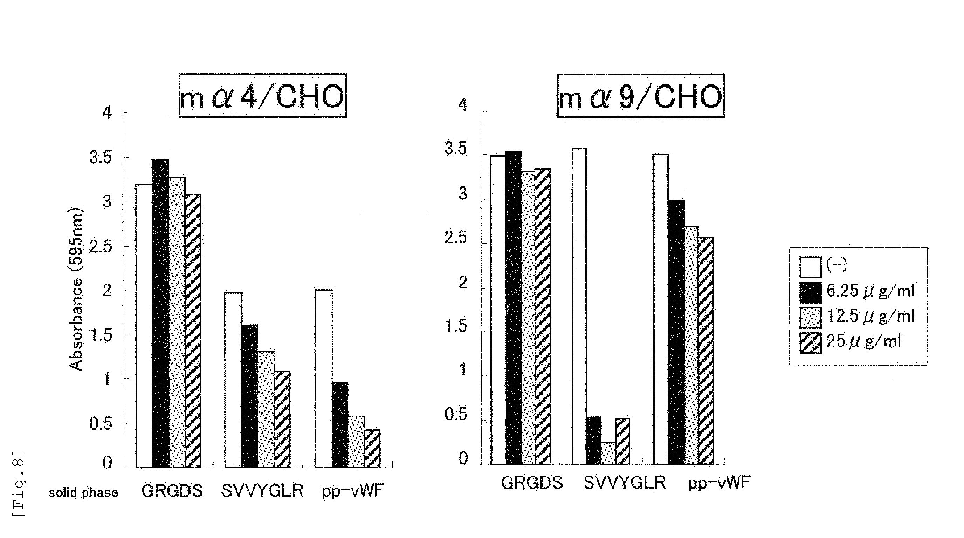

Figure 8 shows a graph of a result of investigation of a integrin α4 and integrin α9 mediated cell adhesion inhibitory function of the 1-2-1 mutant. The vertical axis indicates absorbance at 595 nm to be an index of the number of cells bound to a ligand, and the horizontal axis indicates each ligands used. "mα4/CHO" means that CHO cells expressing mouse integrin α4 were used, and "mα9/CHO" means that CHO cells expressing mouse integrin α9 were used. White bars indicate no addition of the 1-2-1 mutant (-). Black bars, dotted bars, and shaded bars indicate addition of the 1-2-1 mutant in an amount of 6.25 µg/mL, 12.5 µg/mL, and 25 µg/mL, respectively.
Figure 9 is a diagram of a schedule for examining influence of 1-2-1 mutant/GST protein on EAE.

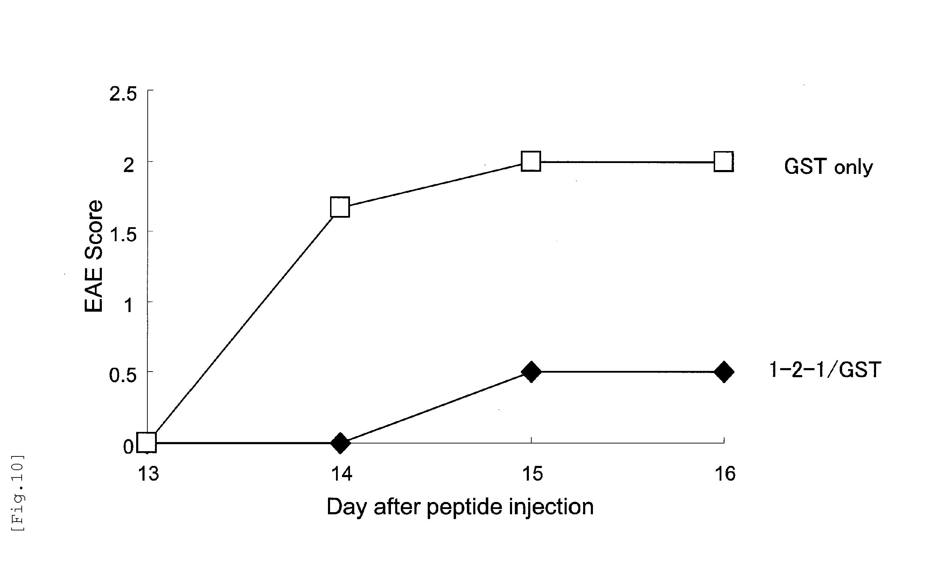

Figure 10 is a graph of a result of examining inhibitory effect of 1-2-1 mutant protein on an EAE. The vertical axis indicates an EAE score, and the horizontal axis indicates the number of days after administration of the 1-2-1 mutant.

### [Description of Embodiments]

### (ITG-α4 mutant)

An ITG-α4 mutant of the present invention can be obtained by referring to the structure (shown in Figure 3) and the sequence (SEQ ID NO: 10) of ITG-α4 and by employing a method known to those skilled in the art, referring to the description of the present application. For example, an ITG-α4 mutant can be obtained by the 3'-race method using a cDNA of cells which are known to express ITG-α4 (for example, T cells such as Jurkat cells, or Rec-1 cells, or the like). It has been reported that ITG-α4 is expressed in bowel, T cells, IgA-producing B cells, (mononuclear) leucocytes and the like. Specifically, a total RNA obtained from cells which are known to express ITG-α4 is extracted by using a QIAGEN RNeasy kit (QIAGEN). From the total RNA, cDNA for the 3'-race method can be synthesized by using Firststrand cDNA synthesis kit (Roche). Specifically, a cDNA for the 3'-race method is synthesized by using a QT primer for the 3'-race method as a primer for reverse transcription (for example, SEQ ID NO: 22). With the obtained cDNA as a template, 1st PCR is performed by using a Q0 primer (for example, SEQ ID NO: 23) and a primer for various regions of ITG-α4 described below (for example, SEQ ID NO: 24, 25, 26 or the like for human ITG-α4). The obtained PCR product is 20 fold diluted and 1 µL of which is used as a template in 2nd PCR, which is performed by using a Q1 primer (for example, SEQ ID NO: 27) and an ITG-α4 primer (for example, SEQ ID NO: 25, 26, 28 or the like for human ITG-α4). The obtained band is purified by gel extraction, the resultant is cloned, and thus, an ITG-α4 mutant can be obtained.

Alternatively, the ITG-α4 mutant of the present invention can be obtained directly as or appropriately modifying a 1-2-1 mutant, a 1-1-2 mutant, a 7-3-2 mutant, a 10-7-3 mutant, a 10-7-2 mutant, or a 9-5-4 mutant. If the 1-2-1 mutant, the 1-1-2 mutant, the 7-3-2 mutant, the 10-7-3 mutant, the 10-7-2 mutant or the 9-5-4 mutant is directly used as the ITG-α4 mutant, it can be prepared in accordance with a method described in Example 2 of the present specification. Furthermore, the modification can be performed by appropriately modifying an amino acid by, for example, substituting, deleting, adding or inserting an amino acid by using a technique usually employed in the field of protein engineering.

Specifically, the ITG-α4 mutant of the present invention can be manufactured by the following method: carrying out PCR with using a cDNA of cells from which the mutant has been obtained as a template, and appropriately designing a primer for amplifying a DNA encoding a desired mutant. The resulting PCR product is digested with restriction enzymes appropriately selected, and then integrated into an expression vector, which is introduced into host cells by using Lipofectamine 2000 (Invitrogen) or the like, and the culture supernatant is purified if necessary. Alternatively, a DNA sequence can be designed from the amino acid sequence and produced by synthesis to be integrated into an expression vector by using a method known to those skilled in the art. Alternatively, a short mutant like the 1-2-1 mutant may be directly manufactured by peptide synthesis.

### (Method for measuring binding activity)

It can be determined whether the ITG-α4 mutant has binding ability to a ligand of ITG-α4 or ITG-α9 by employing a method known to those skilled in the art with referring to the description of the present application. Specifically, it can be determined by using the ligand solid phase ELISA as follows: a ligand of ITG-α4 or ITG-α9 is immobilized, and after blocking with BSA/PBS, a solution containing a test ITG-α4 mutant labeled with FLAG is added thereto. After performing a reaction for 1 hour at 40°C, a mixture of anti-FLAG antibodies and anti-mouse IgGs is added to the resulting solution. Thereafter, a reaction is performed for 30 minutes at 4°C, and the resultant is washed and then color-developed with a TMB solution to analyze the binding ability.

### (Method for measuring activity of binding inhibition)

Whether the ITG-α4 mutant has an activity to inhibit binding of ITG-α4 to its ligand or binding of ITG-α9 to its ligand can be determined by examining whether the ITG-α4 mutant can inhibit binding (cell adhesion) of cells expressing ITG-α4 or ITG-α9 to the ligand thereof. Specifically, BSA is coupled with a ligand of ITG-α4 or a ligand of ITG-α9, and the resultant is incubated overnight at 4°C to immobilize. After blocking for 1 hour with a DMEM medium containing BSA, a mixture of cells (such as CHO cells) expressing integrin α4 or integrin α9 and a GST-fused test ITG-α4 mutant is added thereto, and then cultured in a CO₂ incubator at 37°C for 1 hour. Non-adherent cells were removed by using PBS, and adherent cells are fixed and stained with 20% methanol containing 0.5% crystal violet. The fixed and stained cells are solubilized with 20% acetic acid, and then a cell adhesion inhibitory effect can be measured and quantitatively determined in accordance with absorbance at 595 nm.

### (Pharmaceutical composition)

The ITG-α4 mutant of the present invention can be used as a pharmaceutical composition according to general knowledge in the field of protein medicines which those skilled in the art usually employ. Accordingly, in one aspect, the present invention relates to a pharmaceutical composition containing the ITG-α4 mutant of the present invention as an active ingredient. Specifically, the pharmaceutical composition of the present invention can be used as a therapeutic agent or a preventive agent for cancer, cancer metastasis, multiple myeloma, inflammatory diseases such as rheumatoid arthritis, bronchitis, inflammatory bowel disease, Crohn disease, and multiple sclerosis, or autoimmune disease, acute central nervous system damage, immune disorder such as HIV, allergic encephalomyelitis, hypersensitivity, T cell dependent autoimmune diseases such as type I diabetes, allergic pneumonia, immunocomplex-mediated pneumonopathy, acute nephrotoxic nephritis, delayed hypersensitivity (sclerema and deposition of fibrin), arterial sclerosis and cardiac infarction, or can be used as an inhibitor for rejection in transplantation, such as rejection occurring in vascularized heart allograft.

In using the ITG-α4 mutant of the present invention as a pharmaceutical composition, the administration route can be, for example, oral administration, buccal administration, intratracheal administration, subcutaneous administration, intramuscular administration, intravascular (intravenous) administration or dermal administration. Furthermore, the pharmaceutical composition can be formulated as, for example, injections, capsules, tablets, syrups, granules, and embrocation such as ointments. The ITG-α4 mutant of the present invention can be singly administered, or administered together with a pharmacologically acceptable carrier.

### (Antibody)

An antibody of the present invention can be obtained by the following method: first, an immunogen used for preparing an antibody of the present invention is obtained by transforming an expression vector (such as pGEX (for E. coli) or pcDNA3.1 (for expression in animal cells)) containing a DNA encoding a polypeptide having the whole or a part of the ITG-α4 mutant (preferably an ITG-α4 mutant specific amino acid sequence not present in ITG-α4, such as SEQ ID NOs: 15 to 19) into E. coli, yeast, insect cells, animal cells or the like, and by culturing the transformed host microorganism or culture cells such as E. coli or the like in a suitable medium (such as an LB medium) to express said polypeptide. Alternatively, a peptide chemically synthesized to have such a sequence may be used.

Immunization of an animal by using the above obtained antigen is performed as follows: the above obtained antigen is dissolved in a sodium phosphate buffer solution (PBS), and non-human mammal or bird is immunized with the dissolved solution optionally with an immunostimulant (such as a mineral oil or an aluminum precipitate and heat-killed bacterium or lipopolysaccharide, Freund's complete adjuvant, or Freund's incomplete adjuvant). The immunogen can be administered to an animal, for example, by subcutaneous injection, intraperitoneal injection, intravenous injection, intramuscular injection or sole injection. The amount of immunogen to be used is not especially limited as long as an antibody can be produced, and is preferably 0.1 to 1000 µg, more preferably 1 to 500 µg and even more preferably 10 to 100 µg. The immunization can be performed once or several times at appropriate intervals. Preferably, the immunization can be performed a plurality of times (preferably, 2 to 5 times in total) every 1 to 5 weeks. A polyclonal antibody can be obtained by purification from a serum of an animal showing sufficient antibody titer.

For producing a monoclonal antibody, it can be prepared by fusing antibody producing cells originated from a spleen or the like of an immunologically sensitized animal immunized by the aforementioned method with myeloma cells to obtain hybridoma, and then culturing said hybridoma. The fusion method can be, for example, Milstein's method (Galfre, G. & Milstein, C., Methods Enzymol. 73: 3-46, 1981). The antibody can be obtained by culturing the hybridoma in vitro and purifying the resulting culture medium.

### (Production of human-type chimeric antibody)

When the antibody of the present invention is a human-type chimeric antibody, it can be obtained by following steps: preparing DNAs encoding VH and VL of a nonhuman animal monoclonal antibody capable to bind to the ITG-α4 mutant, connecting the DNAs to a constant region cDNA of human immunoglobulin, inserting the connected DNA into an expression vector, transforming a suitable host cell with the vector, and having the human-type chimeric antibody to be expressed(Morrison, S. L. et al., Proc. Natl. Acad. Sci. USA, 81, 6851-6855, 1984).

### (Production of humanized antibody)

When the antibody of the present invention is a humanized antibody, it can be obtained by following steps: constructing a DNA encoding a V region in which amino acid sequences encoding CDRs of VH and VL of a nonhuman animal monoclonal antibody capable to bind to the ITG-α4 mutant are transplanted into FRs of VH and VL of a human antibody, connecting the constructed DNA to a constant region cDNA of human-derived immunoglobulin, inserting the connected DNA into an expression vector, transforming a suitable host cell with the vector, and having the humanized antibody to be expressed(see L. Rieohmann et al., Nature, 332, 323, 1988: Kettleborough, C. A. et al., Protein Eng., 4, 773-783, 1991: and Clark M., Immunol. Today., 21, 397-402, 2000). CDRs of the non-human animal monoclonal antibody can be determined by comparing an amino acid sequence predicted from the DNA sequence encoding VH and VL of said non-human animal monoclonal antibody obtained as described above with the whole amino acid sequences of VH and VL of a known antibody. Amino acid sequences of the known antibody can be obtained, for example, as amino acid sequences of antibodies registered in database such as Protein Data Bank. The FRs used for the humanized antibody are not especially limited as long as transplanted humanized antibody exerts the effect of the present invention, and are preferably human antibody FRs which give a steric structure of variable region (hereinafter referred to as the "V region") to the humanized antibody that is similar to the steric structure of V region of the non-human animal monoclonal antibody to which CDRs originated, or human antibody FRs having high homology in amino acid sequence to FRs of the non-human animal monoclonal antibody used. A DNA sequence encoding the V region of the humanized antibody used is designed as a DNA sequence corresponding to the amino acid sequence wherein the amino acid sequences of the CDRs of the non-human animal monoclonal antibody are connected to the amino acid sequences of a human antibody FRs. The DNA encoding the V region of the humanized antibody can be prepared based on the designed DNA sequence by using a method known to those skilled in the art.

### (Human antibody)

A human antibody can be obtained by, for example, utilizing a human antibody phage library or a human antibody producing transgenic mouse (Tomizuka et al., Nature Genet., 15, 146-156 (1997)). In using a human antibody phage library, for example, the ITG-α4 mutant or a peptide having an epitope sequence recognized by the antibody of the present invention is immobilized on a solid phase, to which a phage antibody library is reacted, and unbound phages are removed by washing, and then, bound phages are recovered, so as to obtain a desired clone (panning). Furthermore, the obtained phages may be amplified so as to repeatedly perform panning on a library of the amplified phages to improve the accuracy of obtained clone. By analyzing a VH gene and a VL gene of the obtained clone, a complete human antibody having these gene sequences can be produced.

A human antibody producing transgenic mouse is a mouse in which an endogenous immunoglobulin (Ig) gene is knocked out and an Ig gene of a human antibody is introduced. The human antibody producing transgenic mouse can be obtained, for example, by the following method: treating a human-mouse hybrid cell with colcemid (a spindle fiber formation inhibitor) for 48 hours to form a microcell, a structure in which one to a few chromosomes are wrapped with a nuclear membrane, fusing the isolated microcell with a chromosome receiving cell (a mouse ES cell) by using polyethylene glycol in the presence of cytochalasin B to produce a microcell hybrid ES cell, injecting the hybrid ES cell into a mouse germ, immunizing a human antibody producing transgenic mouse with an antigen (preferably, a peptide having an epitope sequence recognized by the antibody of the present invention) according to the above-described method for preparing an anti-ITG-α4 mutant antibody to obtain an anti-ITG-α4 mutant human antibody.

### (Production of antibody fragment)

An F(ab')₂ fragment (an antibody fragment having antigen binding activity and a molecular weight of approximately 100 thousands) can be obtained by treating an IgG antibody of the present invention with pepsin so as to cut at the 234th amino acid residue of an H chain. An Fab' fragment can be obtained by treating the F(ab')₂ fragment obtained as described above with dithiothreitol. Alternatively, an Fab' fragment of the present invention can be obtained from a DNA encoding an Fab' of the antibody of the present invention. An Fab fragment (an antibody fragment having antigen binding activity and a molecular weight of approximately 50 thousands, in which an about half region on the N-terminal side of an H chain and the entire region of an L chain are bound through a disulfide bond) can be obtained by treating the antibody of the present invention with papain so as to cut at the 224th amino acid residue of an H chain. Alternatively, an Fab fragment of the present invention can be obtained from a DNA encoding an Fab of the antibody of the present invention. A scFv can be obtained by inserting a DNA encoding a linker sequence between cDNAs encoding VH and VL of the antibody of the present invention, so as to construct a DNA encoding the scFv. The length of the linker is not especially limited as long as the length is sufficient for associating VH and VL, and is preferably 10 to 20 residues, and more preferably 15 residues. A sequence of the linker is not especially limited as long as it does not inhibit folding of a polypeptide chain of two domains VH and VL, and is preferably a linker consisting of glycine and/or serine, and more preferably GGGGS (G: glycine, S-serine) or a repeat sequence thereof. A dsFv can be obtained by substituting one amino acid residue in each of VH and VL with a cysteine residue, and binding these cysteine residues through a disulfide bond. A diabody can be obtained by constructing a DNA encoding the above-described scFV to have a linker with 8 or less (preferably, 5 residues) amino acid residues. A bispecific diabody can be obtained by producing a scFv by combining DNAs of VHs and VLs of different two scFvs. A peptide containing a CDR of the present invention can be obtained by designing a peptide having an amino acid sequence of a CDR of VH or VL of the antibody of the present invention.

In order to illustrate the present invention in more details, examples are described below, however the present invention is not limited to those in the examples. All literatures cited herein are incorporated by reference in their entirety.

### (Example 1) Identification of integrin α4 mutants

Novel human α4 integrin mutants were identified by the 3'-race method using cDNAs originated from Jurkat cells and Rec-1 cells. A total RNA obtained from human T cell Jurkat cell line (ATCC, TIB-152) and human mantle cell lymphoma Rec-1 cell line (ATCC, CRL-3004) was extracted by using a QIAGEN RNeasy kit (QIAGEN). Specifically, a cDNA for the 3'-race method was synthesized by using a primer for the 3'-race method, a QT primer (5'-CCAGTGAGCAGAGTGACGAGGACTCGAGCTCAAGCTTTTTTTTTTTTTTTTT-3':
SEQ ID NO: 22) as a primer for reverse transcription. With the obtained cDNA used as a template, 1st PCR was performed by using a Q0 primer (5'-CCAGTGAGCAGAGTGACG-3':
SEQ ID NO: 23) and the following three primers for various regions of human integrin α4 (α4-N: 5'-TGTCTCGAGTGGCCGTTTAGTGTTGAATGT-3': SEQ ID NO: 24; α4-1246: 5'-ACTGGTGGTTGCTATGGAGTA-3': SEQ ID NO: 25; α4-2118: 5'-CTTTTCGGTCTGATTCTGCTG-3': SEQ ID NO: 26). The obtained PCR product was diluted by 20 times, and 2nd PCR was performed using 1 µL of said diluted first PCR product as a template. As a primer set for the 2nd PCR, a Q1 primer (5'-GAGGACTCGAGCTCAAGC-3': SEQ ID NO: 27) and three human integrin α4 primers (α4-1246: 5'-ACTGGTGGTTGCTATGGAGTA-3': SEQ ID NO: 25; α4-2118: 5'-CTTTTCGGTCTGATTCTGCTG-3': SEQ ID NO: 26; α4-2556: 5'-CACTTCAGCCAATTCTTCAGC-3': SEQ ID NO: 28) were used. The obtained plural bands were purified by gel extraction, and cloned by using a TOPO cloning kit (Invitrogen), and nucleotide sequences were determined by sequencing.

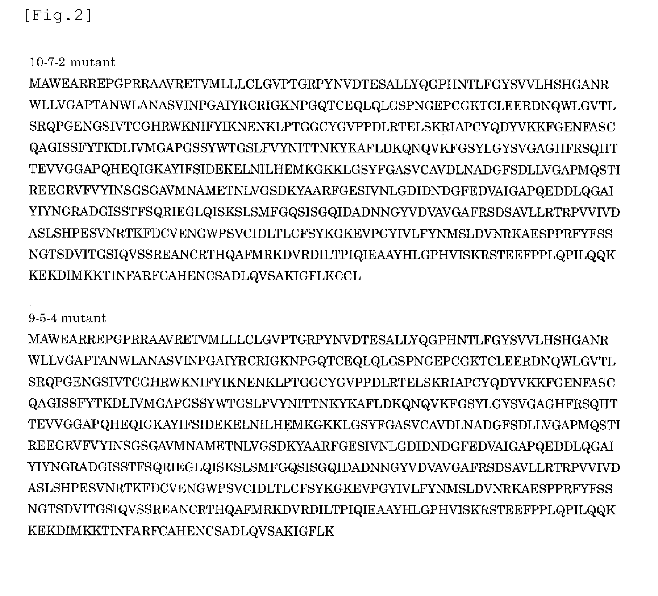

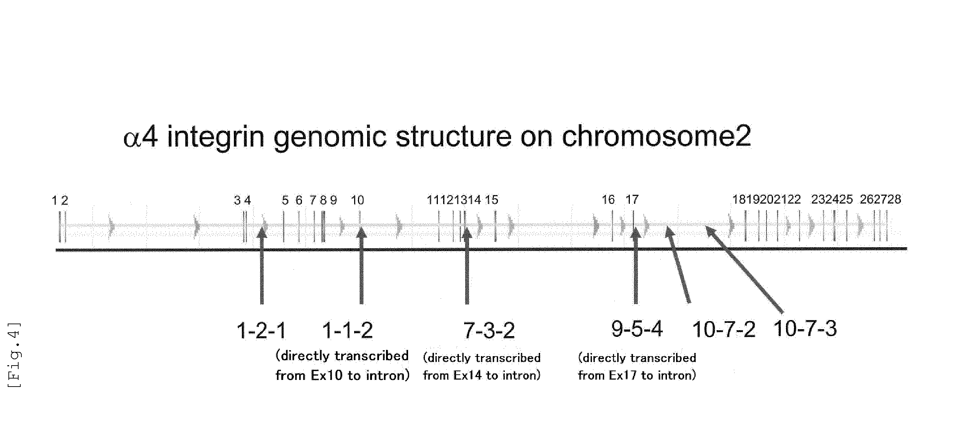

As a result, six novel integrin α4 mutants were successfully identified. The identified novel mutants were respectively designated as the 1-1-2 mutant, the 1-2-1 mutant, the 7-3-2 mutant, the 10-7-3 mutant, the 10-7-2 mutant and the 9-5-4 mutant. Amino acid sequences of the identified six integrin α4 mutants, the 1-2-1 mutant, the 1-1-2 mutant, the 7-3-2 mutant, the 10-7-3 mutant, the 10-7-2 mutant and the 9-5-4 mutant, are shown in Figures 1 and 2. The sequences of these mutants are schematically illustrated in Figure 3, and genomic structures on chromosome 2 of these integrin α4 mutants are illustrated in Figure 4.

### (Example 2) Expression of integrin α4 mutants

All the obtained integrin α4 mutants include only extracellular regions of integrin α4, and therefore, it was presumed that they were secretory proteins. Therefore, in order to express each mutant in culture cells, a plasmid comprising a FLAG tag added to the C-terminal of each mutant was constructed. Expression vectors for the six integrin α4 mutants (the 1-1-2 mutant, the 1-2-1 mutant, the 7-3-2 mutant, the 10-7-3 mutant, the 10-7-2 mutant and the 9-5-4 mutant) were constructed as follows: using cDNAs obtained from Jurkat cells (ATCC, TIB-152) and Rec-1 cells (ATCC, CRL-3004) as templates, PCR was performed with an α4-N primer and an each mutant specific 3'-end primer. The 3'-end primers used are shown below. To each primer, a FLAG tag sequence was added for expression analysis.
ha4-1-1-2-FLAG-RV primer: 5'-TTACTCTAGACTATTTATCGTCATCATCTTTGTAGTCATTACCTTCAAAGCCATCAT T-3' (SEQ ID NO: 29);
ha4-1-2-1-FLAG-RV primer: 5'-TCGTTCTAGACTATTTATCGTCATCATCTTTGTAGTCTGTCCTAGCTCTGTACTTGC T-3' (SEQ ID NO: 30);
ha4-7-3-2-FLAG-RV primer: 5'-CCACTCTAGACTATTTATCGTCATCATCTTTGTAGTCATATTGTAGGGCATACCCAC C-3' (SEQ ID NO: 31);
ha4-10-6-3-FLAG-RV primer: 5'-TTGATCTAGACTATTTATCGTCATCATCTTTGTAGTCTGAGGAAAAGCTGAGAGAGT T-3' (SEQ ID NO: 32);
ha4-10-7-2-FLAG-RV primer: 5'-CCCATCTAGACTATTTATCGTCATCATCTTTGTAGTCGAGACAACACTTCAAAAACC C-3' (SEQ ID NO: 33);
ha4-9-5-4-FLAG-RV primer: 5'-CGTTTCTAGACTATTTATCGTCATCATCTTTGTAGTCCTTCAAAAACCCAATCTTTG C-3' (SEQ ID NO: 34).
Each PCR product was digested with Xhol and Xbal restriction enzymes, and inserted into a pcDNA3.1 expression vector (Invitrogen), and the sequence was determined by sequencing.

The produced expression vector was introduced into COS1 cells (ATCC, CRL-1650) by using Lipofectamine 2000 (Invitrogen), and after 2 days a culture supernatant was concentrated by 10 times with Vivaspin (GE), which was used for performing Western blotting with an anti-FLAG antibody (Wako Pure Chemical Industries, Ltd.).

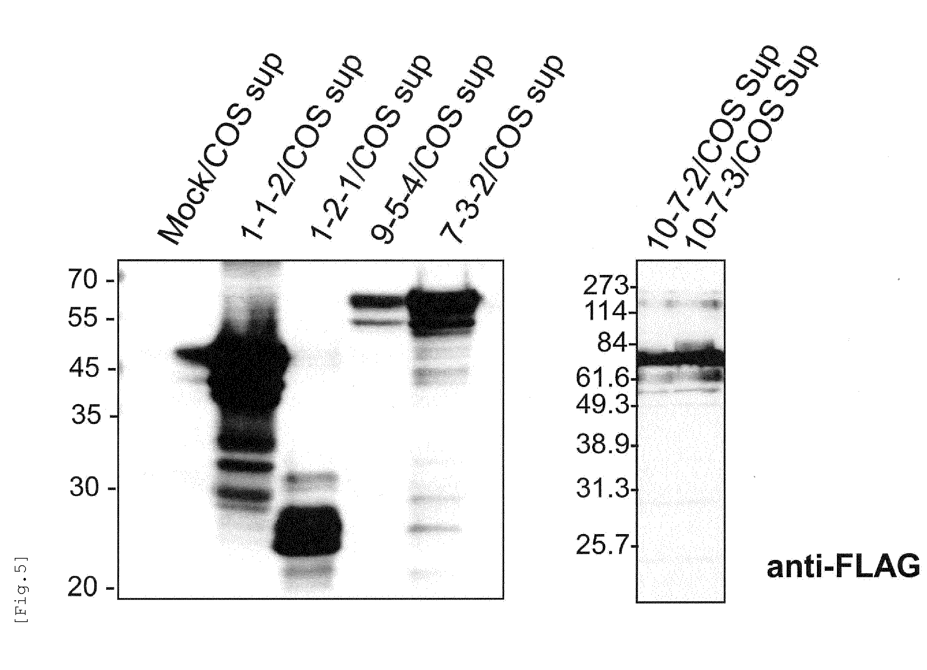

The results of the Western blotting are shown in Figure 5. As illustrated in the picture, it was confirmed that each integrin α4 mutant is secreted into a culture supernatant.

### (Example 3) Binding capacity of integrin α4 mutant to integrin α4 ligand

The binding ability of integrin α4 mutant contained in the supernatant to a ligand of integrin α4 was examined by the ligand solid phase ELISA. 10 µg/mL of a human OPN RAA Nhalf protein as a ligand of integrin α4 (Kon, S., et al., (2002) J. Cell. Biochem., 84: 420-432) or BSA as a control was immobilized at 4°C overnight, and after blocking with 1% BSA/PBS, the same culture supernatant concentrated by 10 times as in Example 2 was added thereto. After incubating at 4°C for 1 hour, a mixture of an anti-FLAG antibody (Wako Pure Chemical Industries, Ltd.) and an anti-mouse IgG (Immuno-bological Laboratories Co., Ltd.) was added, and incubated at 4°C for 30 minutes. After washing, a TMB solution (DAKO) was added to develop color for analyzing binding capacity.

The result is shown in Figure 6. All the mutants other than the 9-5-4 mutant had binding ability to the human OPN RAA Nhalf protein, a ligand of integrin α4. From the result of the Western blotting, it is presumed that this result of the 9-5-4 mutant was not due to lack of its binding ability but due to small amount secreted into the culture medium.

### (Example 4) Analysis of expression of endogenous 1-2-1 mutant

### (1) Production of anti-1-2-1 mutant antibody

A rabbit polyclonal antibody was prepared by using a 1-2-1 mutant specific amino acid sequence GSISKYRART (SEQ ID NO: 15) as an antigen, which was used for analysis of endogenous expression of the 1-2-1 mutant. Specifically, bovine thyroglobulin was introduced as a carrier into the 1-2-1 mutant specific amino acid sequence GSISKYRART (SEQ ID NO: 15), which was immunized a rabbit. The resultant antiserum was purified by using thiol Sepharose beads (GE) to which the GSISKYRART peptide (SEQ ID NO: 15) was bound, so as to obtain an anti-1-2-1 mutant antibody.

### (2) Preparation of culture supernatant

The α4 integrin mutant (1-2-1 mutant) in COS1 cells (ATCC, CRL-1650) was transiently overexpressed by introducing the expression vector of the 1-2-1 mutant prepared in Example 2 into COS1 cells (ATCC, CRL-1650) with Lipofectamine 2000 (Invitrogen). The obtained cells were cultured in a DMEM medium (containing no serum) for 2 days, and a culture supernatant and cells were recovered. The culture supernatant was concentrated by 10 times with Vivaspin (GE), which was used for Western blotting. Jurkat cells (ATCC, TIB-152) and Rec-1 cells (ATCC, CRL-3004) were cultured without a serum in a TIL medium (Immuno-biological Laboratories Co., Ltd.), and a culture supernatant at 3 days was concentrated by 20 times with Vivaspin, which was used for Western blotting.

### (3) Confirmation of expression by Western blotting

The Western blotting was performed, after SDS-PAGE, through transfer to immobilon-P membrane (Millipore Inc.). Since the FLAG tag was added to all the α4 mutants, the expression of the integrin α4 mutant (the 1-2-1 mutant) in COS cells was blotted with an anti-FLAG antibody (Wako Pure Chemical Industries, Ltd.). Expression of the endogenous integrin α4 mutant (1-2-1 mutant) was blotted with the anti-1-2-1 mutant antibody prepared in the aforementioned manner. The luminescence detection was carried out by using ECL-plus (Perkin-Elmer).

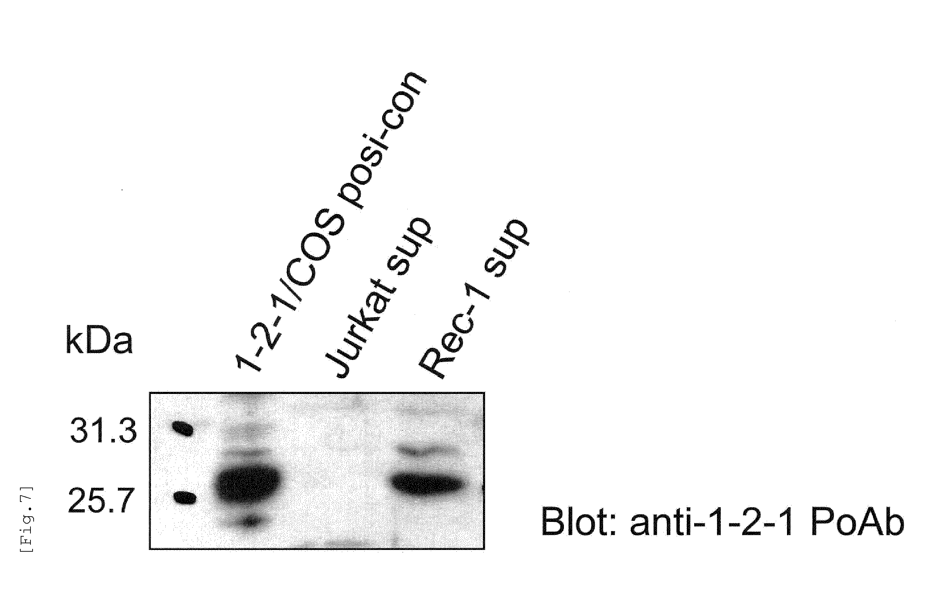

From the results of the Western blotting, it was observed that the Rec-1 cells (ATCC, CRL-3004) sample showed a band at the same position as the culture supernatant of the 1-2-1 mutant transformed COS cells (Figure 7). In other words, it was revealed that the 1-2-1 mutant was endogenously expressed and secreted at a level of protein in the Rec-1 cells.

### (Example 5) Cell adhesion inhibitory function of 1-2-1 mutant via integrin α4 and integrin α9

### (1) Production of GST-fused 1-2-1 mutant protein

In order to analyze the function of the 1-2-1 mutant, the 1-2-1 mutant was expressed as a GST-fused protein in E. coli, and purified by a general method using glutathione Sepharose. For prepare integrin α4 mutant (1-2-1 mutant) protein in E. coli, the 1-2-1 mutant was recombined into pGEX6P-1 (GE). PCR was performed with a 1-2-1 mutant expression vector as a template, and a 5'-end primer (5'-GAGGAATTCTACAACGTGGACACTGAGAG-3': SEQ ID NO: 35) designed to delete an N-terminal signal sequence of the 1-2-1 mutant and a 3'-end primer (5'-CGTCTCGAGTTATGTCCTAGCTCTGTACTTGC-3': SEQ ID NO: 36). The obtained PCR product was digested with EcoRl and Xhol restriction enzymes, and the resultant was inserted into a pGEX6P-1 vector to match a frame of the GST portion. The sequence was confirmed by sequencing. This plasmid was transformed into E. coli, which was grown in an LB medium containing ampicillin at 37°C. During logarithmic growth phase, the E. coli was cultured at 20°C for 1 hour, and then added isopropyl-β-D-1-thiogalactopyranoside (IPTG) to be a final concentration of 0.3 mM, which was cultured at 20°C overnight. The E. coli was recovered and suspended in NETN 150 (20 mM Tris-HCl (pH 8.0), 150 mM NaCl, 1 mM EDTA, 0.5% (v/v) NP-40), and then sonicated by using an ultrasonic grinder. The supernatant after centrifugation was added with glutathione beads (GE, and then rotated at 4°C overnight. After collecting the beads by centrifugation, the resultant was washed with NETN 100 (20 mM Tris-HCl (pH 8.0), 100 mM NaCl, 1 mM EDTA, 0.5% (v/v) NP-40) three times, and eluted with an eluent (20 mM reduced glutathione (Sigma), 100 mM Tris-HCl (pH 8.8)). The obtained GST-fused 1-2-1 mutant protein was dialyzed against PBS and quantitatively determined by using a protein assay kit (Bio-Rad).

### (2) Preparation for CHO cells expressing integrin α4 or integrin α9 (designated as "mα4/CHO" or "mα9/CHO", respectively)

A mouse α4 integrin (herein, designated as "mα4") expression vector was prepared as follows: PCR was performed with a mouse melanoma cell line B16-F10 (ATCC, CRL-6475) as a template and an mα4-Fw primer (5'-CGAGGATCCTGAATGTTCTCCACCAAGAGC-3': SEQ ID NO: 37) and an mα4-RV primer (5'-TTACTCGAGACGGGTCTTCTGAACAGGATT-3': SEQ ID NO: 38), and the obtained PCR product was digested with BamHl and Xhol restriction enzymes, and cloned into a pcDNA3.1 vector (Invitrogen).

A mouse α9 integrin (herein, designated as "mα9") expression vector was constructed as follows: PCR was performed with a cDNA prepared from a mouse placenta as a template and an mα9-Fw primer (5'-GCTAAGCTTCTCTCGACTGTAGCCCATCG-3': SEQ ID NO: 39) and an mα-RV primer (5'-CCATCTAGAGCAACTGCTGAGAGGAAAC-3': SEQ ID NO: 40), and cloned by using a TOPO cloning kit (Invitrogen) (mα9/TOPO). After confirming the sequence of the mouse α9 integrin by sequencing, an mα9 integrin expression vector added with a FLAG tag was prepared by PCR with the mα9/TOPO as a template and the mα9-Fw primer and an mα-FLAG-RV primer (5'-CTGTCTAGATTACTTGTCATCGTCATCCTTGTAGTCCTGGTTTTTCTGGACCCAGTC -3': SEQ ID NO: 41). The obtained PCR product was digested with Hind III and Xbal restriction enzymes, and integrated into a pcDNA 3.1 vector (Invitrogen).

The sequences of the vectors constructed for mouse α4 integrin and α9 integrin were confirmed by sequencing. Respective genes were introduced into CHO-K1 cells (ATCC, CCL-61) by using Lipofectamine 2000 (Invitrogen), and after selection with 10% FCS in a DMEM medium containing 1 mg/mL G418 (PAA) as an antibiotic, the CHO-K1 cells were limiting diluted to confirm expression of mouse α4 integrin and mouse α9 integrin in single colonies. The expression of mouse α4 integrin was confirmed by flow cytometry analysis using an anti-mouse α4 integrin antibody (R1-2) (Biolegend). The expression of mouse α9 integrin was confirmed by Western blotting using an anti-FLAG antibody (Wako Pure Chemical Industries, Ltd.). The clone having the highest level of expression was used for a cell adhesion inhibitory activity test.

### (3) Cell adhesion inhibitory activity test

The obtained purified GST-fused 1-2-1 mutant protein (1-2-1/GST) was used for a cell adhesion inhibitory test. In the cell adhesion test, peptides of functional site of various extracellular matrix proteins, specifically the following peptides, were bound to BSA and were used as various integrin ligands: 5 µg/mL GRGDS (SEQ ID NO: 42) peptide (a peptide for cell adhesion via RGD-dependent integrin such as αv integrin: negative control); 10 µg/mL SVVYGLR (SEQ ID NO: 20) peptide (functional peptide of OPN which exerts cell adhesion activity through binding to integrin α4 and integrin α9); and 10 µg/mL QDHSFSIVIETVQ (SEQ ID NO: 21) peptide (functional peptide of pp-vWF which exerts cell adhesion ability through binding to integrin α4 and integrin α9).

Each functional site peptide of extracellular matrix proteins coupled to BSA was incubated at 4°C overnight for immobilization. After blocking with a DMEM medium containing 0.5% BSA for 1 hour, a mixture of CHO cells expressing either of α4 integrin or α9 integrin and 1-2-1/GST protein (in a final concentration of 6.25 µg/mL, 12.5 µg/mL or 25 µg/mL) was added, which is cultured in a CO₂ incubator at 37°C for 1 hour. Non-adherent cells were removed with PBS. Adherent cells were fixed and stained with 20% methanol containing 0.5% crystal violet, which were solubilized with 20% acetic acid, and absorbance at 595 nm was measured with an immunoreader, so as to examine the cell adhesion inhibitory effect.

### (4) Results

It was observed that the 1-2-1/GST protein did not inhibit RGD-dependent cell adhesion, but concentration dependently inhibited cell adhesion dependent on the SVVYGLR (SEQ ID NO: 20) peptide or the QDHSFSIVIETVQ (SEQ ID NO: 21) peptide which are capable to bind integrin α4 and integrin α9 (Figure 8). In other words, it was found that the 1-2-1/GST protein has a function to simultaneously inhibit cell adhesion via integrin α4 and integrin α9. In addition, the adhesion inhibitory activity against fibronectin EIIIA was measured similarly, and the 1-2-1/GST protein has a function to simultaneously inhibit cell adhesion via integrin α4 and integrin α9 (data not shown). OPN is known to be involved in arterial sclerosis, cardiac infarction, cancer metastasis, and inflammatory diseases. Since the ITG-α4 mutant of the present invention can inhibit OPN mediated adhesions via integrin α4 and integrin α9, it was indicated that the ITG-α4 mutant can be a more effective therapeutic agent for these diseases. A pp-vWF is known to be involved in thrombus, blood platelet function, inflammation and the like. Since the ITG-α4 mutant of the present invention can inhibit pp-vWF mediated adhesion via integrin α4 and integrin α9, it was indicated that the ITG-α4 mutant can be a more effective therapeutic agent for inflammatory diseases (Japanese Patent Laid-Open No. 2005-298336) and the like. A fibronectin EIIIA (also known as EDA) is known to be involved in cancer (Clin. Chim. Acta. (2006) 372: 83-93). Since the ITG-α4 mutant of the present invention can inhibit fibronectin EIIIA mediated adhesion via integrin α4 and integrin α9, it was indicated that the ITG-α4 mutant can be a more effective therapeutic agent for cancer or antimetastatic agent.

### (Example 6) EAE inhibitory effect by integrin α4 mutant 1-2-1 protein

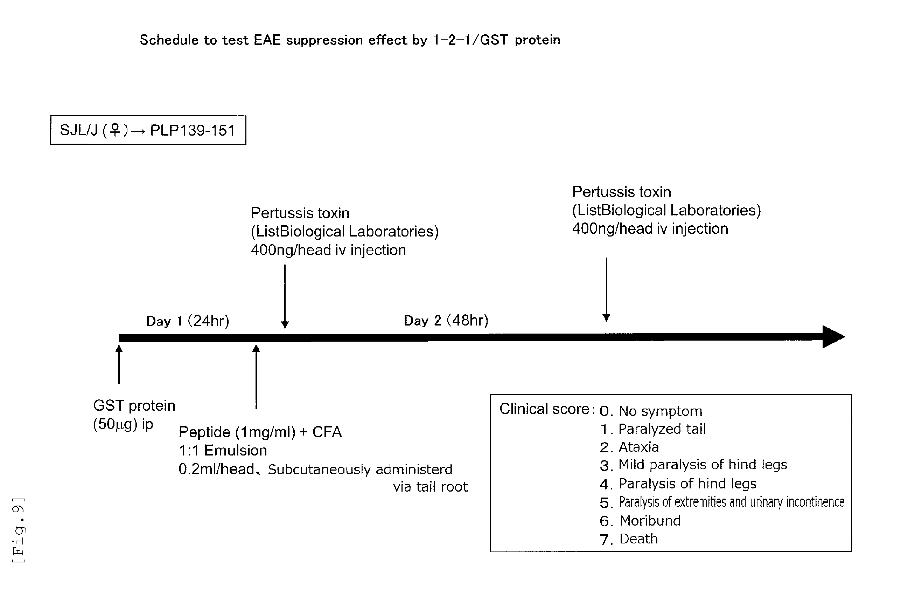

An inhibitory effect for disease exacerbation of the 1-2-1/GST protein was examined by using an EAE model. The EAE was developed as follows: an emulsion of 200 µg of PLP139-151 peptide (sequence: HSLGKWLGHPDKF: SEQ ID NO: 43) and CFA was subcutaneously administered via a tail root of an SJL/J mouse (Charles River Laboratories Japan Inc.), on the same day, pertussis toxin (List Biological Laboratories Inc.) (400 ng) was intravenously injected, after 2 days, pertussis toxin (400 ng) was intravenously injected again. On the day before administering PLP139-151 peptide, the 1-2-1/GST protein or 50µg of GST protein (as a negative control) was intraperitoneally administered. A clinical score for EAE was determined as follows: 0: no symptom; 1: paralyzed tail; 2: ataxia; 3: mild paralysis of hind legs; 4: paralysis of hind legs; 5: paralysis in extremities and urinary incontinence; 6: moribund; and 7: death. A schedule for examining the influence of the 1-2-1/GST protein on EAE is shown in Figure 9.

As a result of the examination, it was found that the 1-2-1/GST protein delays the day of onset (onset), and furthermore, drastically suppresses the EAE score (Figure 9). Since the EAE model is a model of multiple sclerosis, the result of this experiment indicates that the ITG-α4 mutant of the present invention can be used as a therapeutic agent or preventive agent for multiple sclerosis.

## Claims

1. An integrin α4 mutant peptide comprising a part of an extracellular region of human integrin α4.

2. The peptide of claim 1, wherein the extracellular region of human integrin α4 includes an amino acid sequence derived from a β-propellor domain of human integrin α4.

3. The peptide of claim 1, wherein the extracellular region of human integrin α4 consist of a sequence selected from SEQ ID NO: 9 and SEQ ID NOs: 11 to 14.

4. The peptide of any one of claims 1 to 3, wherein a sequence selected from SEQ ID NOs: 15 to 19 is bonded to a C-terminal of the extracellular region of human integrin α4 (SEQ ID NO: 10).

5. A peptide comprising a sequence selected from SEQ ID NOs: 4 to 9.

6. The peptide of any one of claims 1 to 5, which binds to a ligand of integrin α4 and/or inhibitsbinding of integrin α4 to said ligand.

7. The peptide of claim 6, wherein the ligand of integrin α4 is a substance selected from the group consisting of vascular cell adhesion molecule-1, fibronectin, junction adhesion molecule-2, mucosal vascular addressin cell adhesion molecule-1, human lymphocyte expression metalloproteinase-like disintegrinlike cysteine rich protein family member L, von Willebrand factor and osteopontin.

8. The peptide of claim 6, wherein the ligand of integrin α4 is von Willebrand factor or OPN.

9. The peptide of claim 6, wherein the ligand of integrin α4 is a peptide comprising an amino acid sequence of SEQ ID NO: 20 or 21.

10. The peptide of any one of claims 1 to 9, which further binds to a ligand of integrin α9 and/or inhibits binding of integrin α9 to said ligand.

11. The peptide of claim 10, wherein the ligand of integrin α9 is a substance selected from the group consisting of vascular cell adhesion molecule-1, fibronectin, junction adhesion molecule-2, mucosal vascular addressin cell adhesion molecule-1, human lymphocyte expression metalloproteinase-like disintegrinlike cysteine rich protein family member L, von Willebrand factor and osteopontin.

12. The peptide of claim 10, wherein the ligand of integrin α9 is von Willebrand factor or OPN.

13. The peptide of claim 10, wherein the ligand of integrin α9 is a peptide comprising an amino acid sequence of SEQ ID NO: 20 or 21.

14. The peptide of any one of claims 1 to 13, which is a soluble peptide.

15. A pharmaceutical composition comprising the peptide of any one of claims 1 to 14 as an active ingredient.

16. An antibody that specifically recognizes the peptide of any one of claims 1 to 14.

17. The antibody of claim 16, which does not recognize integrin α4.


SEQUENCE LISTING

<110>  IMMUNO-BIOLOGICAL LABOLATORIES CO., LTD

<120>  Soluble alpha 4 integrin mutant

<130>  PW12014IB

<150>  JP2011-146164
<151>  2011-06-30

<160>  43

<170>  PatentIn version 3.1

<210>  1
<211>  3
<212>  PRT
<213>  Artificial

<220>
<223>  integrin binding sequence

<400>  1

Arg Gly Asp
1

<210>  2
<211>  3
<212>  PRT
<213>  Artificial

<220>
<223>  integring binding sequence

<400>  2

Leu Asp Val
1

<210>  3
<211>  6
<212>  PRT
<213>  Artificial

<220>
<223>  integrin binding sequence

<400>  3

Gln Ile Asp Ser Pro Leu
1               5

<210>  4
<211>  195
<212>  PRT
<213>  Homo sapiens

<400>  4

Met Ala Trp Glu Ala Arg Arg Glu Pro Gly Pro Arg Arg Ala Ala Val
1               5                   10                  15

Arg Glu Thr Val Met Leu Leu Leu Cys Leu Gly Val Pro Thr Gly Arg
            20                  25                  30

Pro Tyr Asn Val Asp Thr Glu Ser Ala Leu Leu Tyr Gln Gly Pro His
        35                  40                  45

Asn Thr Leu Phe Gly Tyr Ser Val Val Leu His Ser His Gly Ala Asn
    50                  55                  60

Arg Trp Leu Leu Val Gly Ala Pro Thr Ala Asn Trp Leu Ala Asn Ala
65                  70                  75                  80

Ser Val Ile Asn Pro Gly Ala Ile Tyr Arg Cys Arg Ile Gly Lys Asn
                85                  90                  95

Pro Gly Gln Thr Cys Glu Gln Leu Gln Leu Gly Ser Pro Asn Gly Glu
            100                 105                 110

Pro Cys Gly Lys Thr Cys Leu Glu Glu Arg Asp Asn Gln Trp Leu Gly
        115                 120                 125

Val Thr Leu Ser Arg Gln Pro Gly Glu Asn Gly Ser Ile Val Thr Cys
    130                 135                 140

Gly His Arg Trp Lys Asn Ile Phe Tyr Ile Lys Asn Glu Asn Lys Leu
145                 150                 155                 160

Pro Thr Gly Gly Cys Tyr Gly Val Pro Pro Asp Leu Arg Thr Glu Leu
                165                 170                 175

Ser Lys Arg Ile Ala Pro Cys Tyr Gln Gly Ser Ile Ser Lys Tyr Arg
            180                 185                 190

Ala Arg Thr
        195

<210>  5
<211>  386
<212>  PRT
<213>  Homo sapiens

<400>  5

Met Ala Trp Glu Ala Arg Arg Glu Pro Gly Pro Arg Arg Ala Ala Val
1               5                   10                  15

Arg Glu Thr Val Met Leu Leu Leu Cys Leu Gly Val Pro Thr Gly Arg
            20                  25                  30

Pro Tyr Asn Val Asp Thr Glu Ser Ala Leu Leu Tyr Gln Gly Pro His
        35                  40                  45

Asn Thr Leu Phe Gly Tyr Ser Val Val Leu His Ser His Gly Ala Asn
    50                  55                  60

Arg Trp Leu Leu Val Gly Ala Pro Thr Ala Asn Trp Leu Ala Asn Ala
65                  70                  75                  80

Ser Val Ile Asn Pro Gly Ala Ile Tyr Arg Cys Arg Ile Gly Lys Asn
                85                  90                  95

Pro Gly Gln Thr Cys Glu Gln Leu Gln Leu Gly Ser Pro Asn Gly Glu
            100                 105                 110

Pro Cys Gly Lys Thr Cys Leu Glu Glu Arg Asp Asn Gln Trp Leu Gly
        115                 120                 125

Val Thr Leu Ser Arg Gln Pro Gly Glu Asn Gly Ser Ile Val Thr Cys
    130                 135                 140

Gly His Arg Trp Lys Asn Ile Phe Tyr Ile Lys Asn Glu Asn Lys Leu
145                 150                 155                 160

Pro Thr Gly Gly Cys Tyr Gly Val Pro Pro Asp Leu Arg Thr Glu Leu
                165                 170                 175

Ser Lys Arg Ile Ala Pro Cys Tyr Gln Asp Tyr Val Lys Lys Phe Gly
            180                 185                 190

Glu Asn Phe Ala Ser Cys Gln Ala Gly Ile Ser Ser Phe Tyr Thr Lys
        195                 200                 205

Asp Leu Ile Val Met Gly Ala Pro Gly Ser Ser Tyr Trp Thr Gly Ser
    210                 215                 220

Leu Phe Val Tyr Asn Ile Thr Thr Asn Lys Tyr Lys Ala Phe Leu Asp
225                 230                 235                 240

Lys Gln Asn Gln Val Lys Phe Gly Ser Tyr Leu Gly Tyr Ser Val Gly
                245                 250                 255

Ala Gly His Phe Arg Ser Gln His Thr Thr Glu Val Val Gly Gly Ala
            260                 265                 270

Pro Gln His Glu Gln Ile Gly Lys Ala Tyr Ile Phe Ser Ile Asp Glu
        275                 280                 285

Lys Glu Leu Asn Ile Leu His Glu Met Lys Gly Lys Lys Leu Gly Ser
    290                 295                 300

Tyr Phe Gly Ala Ser Val Cys Ala Val Asp Leu Asn Ala Asp Gly Phe
305                 310                 315                 320

Ser Asp Leu Leu Val Gly Ala Pro Met Gln Ser Thr Ile Arg Glu Glu
                325                 330                 335

Gly Arg Val Phe Val Tyr Ile Asn Ser Gly Ser Gly Ala Val Met Asn
            340                 345                 350

Ala Met Glu Thr Asn Leu Val Gly Ser Asp Lys Tyr Ala Ala Arg Phe
        355                 360                 365

Gly Glu Ser Ile Val Asn Leu Gly Asp Ile Asp Asn Asp Gly Phe Glu
    370                 375                 380

Gly Asn
385

<210>  6
<211>  520
<212>  PRT
<213>  Homo sapiens

<400>  6

Met Ala Trp Glu Ala Arg Arg Glu Pro Gly Pro Arg Arg Ala Ala Val
1               5                   10                  15

Arg Glu Thr Val Met Leu Leu Leu Cys Leu Gly Val Pro Thr Gly Arg
            20                  25                  30

Pro Tyr Asn Val Asp Thr Glu Ser Ala Leu Leu Tyr Gln Gly Pro His
        35                  40                  45

Asn Thr Leu Phe Gly Tyr Ser Val Val Leu His Ser His Gly Ala Asn
    50                  55                  60

Arg Trp Leu Leu Val Gly Ala Pro Thr Ala Asn Trp Leu Ala Asn Ala
65                  70                  75                  80

Ser Val Ile Asn Pro Gly Ala Ile Tyr Arg Cys Arg Ile Gly Lys Asn
                85                  90                  95

Pro Gly Gln Thr Cys Glu Gln Leu Gln Leu Gly Ser Pro Asn Gly Glu
            100                 105                 110

Pro Cys Gly Lys Thr Cys Leu Glu Glu Arg Asp Asn Gln Trp Leu Gly
        115                 120                 125

Val Thr Leu Ser Arg Gln Pro Gly Glu Asn Gly Ser Ile Val Thr Cys
    130                 135                 140

Gly His Arg Trp Lys Asn Ile Phe Tyr Ile Lys Asn Glu Asn Lys Leu
145                 150                 155                 160

Pro Thr Gly Gly Cys Tyr Gly Val Pro Pro Asp Leu Arg Thr Glu Leu
                165                 170                 175

Ser Lys Arg Ile Ala Pro Cys Tyr Gln Asp Tyr Val Lys Lys Phe Gly
            180                 185                 190

Glu Asn Phe Ala Ser Cys Gln Ala Gly Ile Ser Ser Phe Tyr Thr Lys
        195                 200                 205

Asp Leu Ile Val Met Gly Ala Pro Gly Ser Ser Tyr Trp Thr Gly Ser
    210                 215                 220

Leu Phe Val Tyr Asn Ile Thr Thr Asn Lys Tyr Lys Ala Phe Leu Asp
225                 230                 235                 240

Lys Gln Asn Gln Val Lys Phe Gly Ser Tyr Leu Gly Tyr Ser Val Gly
                245                 250                 255

Ala Gly His Phe Arg Ser Gln His Thr Thr Glu Val Val Gly Gly Ala
            260                 265                 270

Pro Gln His Glu Gln Ile Gly Lys Ala Tyr Ile Phe Ser Ile Asp Glu
        275                 280                 285

Lys Glu Leu Asn Ile Leu His Glu Met Lys Gly Lys Lys Leu Gly Ser
    290                 295                 300

Tyr Phe Gly Ala Ser Val Cys Ala Val Asp Leu Asn Ala Asp Gly Phe
305                 310                 315                 320

Ser Asp Leu Leu Val Gly Ala Pro Met Gln Ser Thr Ile Arg Glu Glu
                325                 330                 335

Gly Arg Val Phe Val Tyr Ile Asn Ser Gly Ser Gly Ala Val Met Asn

340                 345                 350

Ala Met Glu Thr Asn Leu Val Gly Ser Asp Lys Tyr Ala Ala Arg Phe
        355                 360                 365

Gly Glu Ser Ile Val Asn Leu Gly Asp Ile Asp Asn Asp Gly Phe Glu
    370                 375                 380

Asp Val Ala Ile Gly Ala Pro Gln Glu Asp Asp Leu Gln Gly Ala Ile
385                 390                 395                 400

Tyr Ile Tyr Asn Gly Arg Ala Asp Gly Ile Ser Ser Thr Phe Ser Gln
                405                 410                 415

Arg Ile Glu Gly Leu Gln Ile Ser Lys Ser Leu Ser Met Phe Gly Gln
            420                 425                 430

Ser Ile Ser Gly Gln Ile Asp Ala Asp Asn Asn Gly Tyr Val Asp Val
        435                 440                 445

Ala Val Gly Ala Phe Arg Ser Asp Ser Ala Val Leu Leu Arg Thr Arg
    450                 455                 460

Pro Val Val Ile Val Asp Ala Ser Leu Ser His Pro Glu Ser Val Asn
465                 470                 475                 480

Arg Thr Lys Phe Asp Cys Val Glu Asn Gly Trp Pro Ser Val Cys Ile
                485                 490                 495

Asp Leu Thr Leu Cys Phe Ser Tyr Lys Gly Lys Glu Val Pro Gly Tyr
            500                 505                 510

Ile Gly Gly Tyr Ala Leu Gln Tyr
        515                 520

<210>  7
<211>  647
<212>  PRT
<213>  Homo sapiens

<400>  7

Met Ala Trp Glu Ala Arg Arg Glu Pro Gly Pro Arg Arg Ala Ala Val
1               5                   10                  15

Arg Glu Thr Val Met Leu Leu Leu Cys Leu Gly Val Pro Thr Gly Arg
            20                  25                  30

Pro Tyr Asn Val Asp Thr Glu Ser Ala Leu Leu Tyr Gln Gly Pro His

35                  40                  45

Asn Thr Leu Phe Gly Tyr Ser Val Val Leu His Ser His Gly Ala Asn
    50                  55                  60

Arg Trp Leu Leu Val Gly Ala Pro Thr Ala Asn Trp Leu Ala Asn Ala
65                  70                  75                  80

Ser Val Ile Asn Pro Gly Ala Ile Tyr Arg Cys Arg Ile Gly Lys Asn
                85                  90                  95

Pro Gly Gln Thr Cys Glu Gln Leu Gln Leu Gly Ser Pro Asn Gly Glu
            100                 105                 110

Pro Cys Gly Lys Thr Cys Leu Glu Glu Arg Asp Asn Gln Trp Leu Gly
        115                 120                 125

Val Thr Leu Ser Arg Gln Pro Gly Glu Asn Gly Ser Ile Val Thr Cys
    130                 135                 140

Gly His Arg Trp Lys Asn Ile Phe Tyr Ile Lys Asn Glu Asn Lys Leu
145                 150                 155                 160

Pro Thr Gly Gly Cys Tyr Gly Val Pro Pro Asp Leu Arg Thr Glu Leu
                165                 170                 175

Ser Lys Arg Ile Ala Pro Cys Tyr Gln Asp Tyr Val Lys Lys Phe Gly
            180                 185                 190

Glu Asn Phe Ala Ser Cys Gln Ala Gly Ile Ser Ser Phe Tyr Thr Lys
        195                 200                 205

Asp Leu Ile Val Met Gly Ala Pro Gly Ser Ser Tyr Trp Thr Gly Ser
    210                 215                 220

Leu Phe Val Tyr Asn Ile Thr Thr Asn Lys Tyr Lys Ala Phe Leu Asp
225                 230                 235                 240

Lys Gln Asn Gln Val Lys Phe Gly Ser Tyr Leu Gly Tyr Ser Val Gly
                245                 250                 255

Ala Gly His Phe Arg Ser Gln His Thr Thr Glu Val Val Gly Gly Ala
            260                 265                 270

Pro Gln His Glu Gln Ile Gly Lys Ala Tyr Ile Phe Ser Ile Asp Glu
        275                 280                 285

Lys Glu Leu Asn Ile Leu His Glu Met Lys Gly Lys Lys Leu Gly Ser
    290                 295                 300

Tyr Phe Gly Ala Ser Val Cys Ala Val Asp Leu Asn Ala Asp Gly Phe
305                 310                 315                 320

Ser Asp Leu Leu Val Gly Ala Pro Met Gln Ser Thr Ile Arg Glu Glu
                325                 330                 335

Gly Arg Val Phe Val Tyr Ile Asn Ser Gly Ser Gly Ala Val Met Asn
            340                 345                 350

Ala Met Glu Thr Asn Leu Val Gly Ser Asp Lys Tyr Ala Ala Arg Phe
        355                 360                 365

Gly Glu Ser Ile Val Asn Leu Gly Asp Ile Asp Asn Asp Gly Phe Glu
    370                 375                 380

Asp Val Ala Ile Gly Ala Pro Gln Glu Asp Asp Leu Gln Gly Ala Ile
385                 390                 395                 400

Tyr Ile Tyr Asn Gly Arg Ala Asp Gly Ile Ser Ser Thr Phe Ser Gln
                405                 410                 415

Arg Ile Glu Gly Leu Gln Ile Ser Lys Ser Leu Ser Met Phe Gly Gln
            420                 425                 430

Ser Ile Ser Gly Gln Ile Asp Ala Asp Asn Asn Gly Tyr Val Asp Val
        435                 440                 445

Ala Val Gly Ala Phe Arg Ser Asp Ser Ala Val Leu Leu Arg Thr Arg
    450                 455                 460

Pro Val Val Ile Val Asp Ala Ser Leu Ser His Pro Glu Ser Val Asn
465                 470                 475                 480

Arg Thr Lys Phe Asp Cys Val Glu Asn Gly Trp Pro Ser Val Cys Ile
                485                 490                 495

Asp Leu Thr Leu Cys Phe Ser Tyr Lys Gly Lys Glu Val Pro Gly Tyr
            500                 505                 510

Ile Val Leu Phe Tyr Asn Met Ser Leu Asp Val Asn Arg Lys Ala Glu
        515                 520                 525

Ser Pro Pro Arg Phe Tyr Phe Ser Ser Asn Gly Thr Ser Asp Val Ile
    530                 535                 540

Thr Gly Ser Ile Gln Val Ser Ser Arg Glu Ala Asn Cys Arg Thr His
545                 550                 555                 560

Gln Ala Phe Met Arg Lys Asp Val Arg Asp Ile Leu Thr Pro Ile Gln
                565                 570                 575

Ile Glu Ala Ala Tyr His Leu Gly Pro His Val Ile Ser Lys Arg Ser
            580                 585                 590

Thr Glu Glu Phe Pro Pro Leu Gln Pro Ile Leu Gln Gln Lys Lys Glu
        595                 600                 605

Lys Asp Ile Met Lys Lys Thr Ile Asn Phe Ala Arg Phe Cys Ala His
    610                 615                 620

Glu Asn Cys Ser Ala Asp Leu Gln Val Ser Ala Lys Ile Gly Phe Leu
625                 630                 635                 640

Asn Ser Leu Ser Phe Ser Ser
                645

<210>  8
<211>  644
<212>  PRT
<213>  Homo sapiens

<400>  8

Met Ala Trp Glu Ala Arg Arg Glu Pro Gly Pro Arg Arg Ala Ala Val
1               5                   10                  15

Arg Glu Thr Val Met Leu Leu Leu Cys Leu Gly Val Pro Thr Gly Arg
            20                  25                  30

Pro Tyr Asn Val Asp Thr Glu Ser Ala Leu Leu Tyr Gln Gly Pro His
        35                  40                  45

Asn Thr Leu Phe Gly Tyr Ser Val Val Leu His Ser His Gly Ala Asn
    50                  55                  60

Arg Trp Leu Leu Val Gly Ala Pro Thr Ala Asn Trp Leu Ala Asn Ala
65                  70                  75                  80

Ser Val Ile Asn Pro Gly Ala Ile Tyr Arg Cys Arg Ile Gly Lys Asn
                85                  90                  95

Pro Gly Gln Thr Cys Glu Gln Leu Gln Leu Gly Ser Pro Asn Gly Glu
            100                 105                 110

Pro Cys Gly Lys Thr Cys Leu Glu Glu Arg Asp Asn Gln Trp Leu Gly
        115                 120                 125

Val Thr Leu Ser Arg Gln Pro Gly Glu Asn Gly Ser Ile Val Thr Cys
    130                 135                 140

Gly His Arg Trp Lys Asn Ile Phe Tyr Ile Lys Asn Glu Asn Lys Leu
145                 150                 155                 160

Pro Thr Gly Gly Cys Tyr Gly Val Pro Pro Asp Leu Arg Thr Glu Leu
                165                 170                 175

Ser Lys Arg Ile Ala Pro Cys Tyr Gln Asp Tyr Val Lys Lys Phe Gly
            180                 185                 190

Glu Asn Phe Ala Ser Cys Gln Ala Gly Ile Ser Ser Phe Tyr Thr Lys
        195                 200                 205

Asp Leu Ile Val Met Gly Ala Pro Gly Ser Ser Tyr Trp Thr Gly Ser
    210                 215                 220

Leu Phe Val Tyr Asn Ile Thr Thr Asn Lys Tyr Lys Ala Phe Leu Asp
225                 230                 235                 240

Lys Gln Asn Gln Val Lys Phe Gly Ser Tyr Leu Gly Tyr Ser Val Gly
                245                 250                 255

Ala Gly His Phe Arg Ser Gln His Thr Thr Glu Val Val Gly Gly Ala
            260                 265                 270

Pro Gln His Glu Gln Ile Gly Lys Ala Tyr Ile Phe Ser Ile Asp Glu
        275                 280                 285

Lys Glu Leu Asn Ile Leu His Glu Met Lys Gly Lys Lys Leu Gly Ser
    290                 295                 300

Tyr Phe Gly Ala Ser Val Cys Ala Val Asp Leu Asn Ala Asp Gly Phe
305                 310                 315                 320

Ser Asp Leu Leu Val Gly Ala Pro Met Gln Ser Thr Ile Arg Glu Glu
                325                 330                 335

Gly Arg Val Phe Val Tyr Ile Asn Ser Gly Ser Gly Ala Val Met Asn
            340                 345                 350

Ala Met Glu Thr Asn Leu Val Gly Ser Asp Lys Tyr Ala Ala Arg Phe
        355                 360                 365

Gly Glu Ser Ile Val Asn Leu Gly Asp Ile Asp Asn Asp Gly Phe Glu
    370                 375                 380

Asp Val Ala Ile Gly Ala Pro Gln Glu Asp Asp Leu Gln Gly Ala Ile
385                 390                 395                 400

Tyr Ile Tyr Asn Gly Arg Ala Asp Gly Ile Ser Ser Thr Phe Ser Gln
                405                 410                 415

Arg Ile Glu Gly Leu Gln Ile Ser Lys Ser Leu Ser Met Phe Gly Gln
            420                 425                 430

Ser Ile Ser Gly Gln Ile Asp Ala Asp Asn Asn Gly Tyr Val Asp Val
        435                 440                 445

Ala Val Gly Ala Phe Arg Ser Asp Ser Ala Val Leu Leu Arg Thr Arg
    450                 455                 460

Pro Val Val Ile Val Asp Ala Ser Leu Ser His Pro Glu Ser Val Asn
465                 470                 475                 480

Arg Thr Lys Phe Asp Cys Val Glu Asn Gly Trp Pro Ser Val Cys Ile
                485                 490                 495

Asp Leu Thr Leu Cys Phe Ser Tyr Lys Gly Lys Glu Val Pro Gly Tyr
            500                 505                 510

Ile Val Leu Phe Tyr Asn Met Ser Leu Asp Val Asn Arg Lys Ala Glu
        515                 520                 525

Ser Pro Pro Arg Phe Tyr Phe Ser Ser Asn Gly Thr Ser Asp Val Ile
    530                 535                 540

Thr Gly Ser Ile Gln Val Ser Ser Arg Glu Ala Asn Cys Arg Thr His
545                 550                 555                 560

Gln Ala Phe Met Arg Lys Asp Val Arg Asp Ile Leu Thr Pro Ile Gln
                565                 570                 575

Ile Glu Ala Ala Tyr His Leu Gly Pro His Val Ile Ser Lys Arg Ser
            580                 585                 590

Thr Glu Glu Phe Pro Pro Leu Gln Pro Ile Leu Gln Gln Lys Lys Glu
        595                 600                 605

Lys Asp Ile Met Lys Lys Thr Ile Asn Phe Ala Arg Phe Cys Ala His

610                 615                 620

Glu Asn Cys Ser Ala Asp Leu Gln Val Ser Ala Lys Ile Gly Phe Leu
625                 630                 635                 640

Lys Cys Cys Leu

<210>  9
<211>  641
<212>  PRT
<213>  Homo sapiens

<400>  9

Met Ala Trp Glu Ala Arg Arg Glu Pro Gly Pro Arg Arg Ala Ala Val
1               5                   10                  15

Arg Glu Thr Val Met Leu Leu Leu Cys Leu Gly Val Pro Thr Gly Arg
            20                  25                  30

Pro Tyr Asn Val Asp Thr Glu Ser Ala Leu Leu Tyr Gln Gly Pro His
        35                  40                  45

Asn Thr Leu Phe Gly Tyr Ser Val Val Leu His Ser His Gly Ala Asn
    50                  55                  60

Arg Trp Leu Leu Val Gly Ala Pro Thr Ala Asn Trp Leu Ala Asn Ala
65                  70                  75                  80

Ser Val Ile Asn Pro Gly Ala Ile Tyr Arg Cys Arg Ile Gly Lys Asn
                85                  90                  95

Pro Gly Gln Thr Cys Glu Gln Leu Gln Leu Gly Ser Pro Asn Gly Glu
            100                 105                 110

Pro Cys Gly Lys Thr Cys Leu Glu Glu Arg Asp Asn Gln Trp Leu Gly
        115                 120                 125

Val Thr Leu Ser Arg Gln Pro Gly Glu Asn Gly Ser Ile Val Thr Cys
    130                 135                 140

Gly His Arg Trp Lys Asn Ile Phe Tyr Ile Lys Asn Glu Asn Lys Leu
145                 150                 155                 160

Pro Thr Gly Gly Cys Tyr Gly Val Pro Pro Asp Leu Arg Thr Glu Leu
                165                 170                 175

Ser Lys Arg Ile Ala Pro Cys Tyr Gln Asp Tyr Val Lys Lys Phe Gly

180                 185                 190

Glu Asn Phe Ala Ser Cys Gln Ala Gly Ile Ser Ser Phe Tyr Thr Lys
        195                 200                 205

Asp Leu Ile Val Met Gly Ala Pro Gly Ser Ser Tyr Trp Thr Gly Ser
    210                 215                 220

Leu Phe Val Tyr Asn Ile Thr Thr Asn Lys Tyr Lys Ala Phe Leu Asp
225                 230                 235                 240

Lys Gln Asn Gln Val Lys Phe Gly Ser Tyr Leu Gly Tyr Ser Val Gly
                245                 250                 255

Ala Gly His Phe Arg Ser Gln His Thr Thr Glu Val Val Gly Gly Ala
            260                 265                 270

Pro Gln His Glu Gln Ile Gly Lys Ala Tyr Ile Phe Ser Ile Asp Glu
        275                 280                 285

Lys Glu Leu Asn Ile Leu His Glu Met Lys Gly Lys Lys Leu Gly Ser
    290                 295                 300

Tyr Phe Gly Ala Ser Val Cys Ala Val Asp Leu Asn Ala Asp Gly Phe
305                 310                 315                 320

Ser Asp Leu Leu Val Gly Ala Pro Met Gln Ser Thr Ile Arg Glu Glu
                325                 330                 335

Gly Arg Val Phe Val Tyr Ile Asn Ser Gly Ser Gly Ala Val Met Asn
            340                 345                 350

Ala Met Glu Thr Asn Leu Val Gly Ser Asp Lys Tyr Ala Ala Arg Phe
        355                 360                 365

Gly Glu Ser Ile Val Asn Leu Gly Asp Ile Asp Asn Asp Gly Phe Glu
    370                 375                 380

Asp Val Ala Ile Gly Ala Pro Gln Glu Asp Asp Leu Gln Gly Ala Ile
385                 390                 395                 400

Tyr Ile Tyr Asn Gly Arg Ala Asp Gly Ile Ser Ser Thr Phe Ser Gln
                405                 410                 415

Arg Ile Glu Gly Leu Gln Ile Ser Lys Ser Leu Ser Met Phe Gly Gln
            420                 425                 430

Ser Ile Ser Gly Gln Ile Asp Ala Asp Asn Asn Gly Tyr Val Asp Val
        435                 440                 445

Ala Val Gly Ala Phe Arg Ser Asp Ser Ala Val Leu Leu Arg Thr Arg
    450                 455                 460

Pro Val Val Ile Val Asp Ala Ser Leu Ser His Pro Glu Ser Val Asn
465                 470                 475                 480

Arg Thr Lys Phe Asp Cys Val Glu Asn Gly Trp Pro Ser Val Cys Ile
                485                 490                 495

Asp Leu Thr Leu Cys Phe Ser Tyr Lys Gly Lys Glu Val Pro Gly Tyr
            500                 505                 510

Ile Val Leu Phe Tyr Asn Met Ser Leu Asp Val Asn Arg Lys Ala Glu
        515                 520                 525

Ser Pro Pro Arg Phe Tyr Phe Ser Ser Asn Gly Thr Ser Asp Val Ile
    530                 535                 540

Thr Gly Ser Ile Gln Val Ser Ser Arg Glu Ala Asn Cys Arg Thr His
545                 550                 555                 560

Gln Ala Phe Met Arg Lys Asp Val Arg Asp Ile Leu Thr Pro Ile Gln
                565                 570                 575

Ile Glu Ala Ala Tyr His Leu Gly Pro His Val Ile Ser Lys Arg Ser
            580                 585                 590

Thr Glu Glu Phe Pro Pro Leu Gln Pro Ile Leu Gln Gln Lys Lys Glu
        595                 600                 605

Lys Asp Ile Met Lys Lys Thr Ile Asn Phe Ala Arg Phe Cys Ala His
    610                 615                 620

Glu Asn Cys Ser Ala Asp Leu Gln Val Ser Ala Lys Ile Gly Phe Leu
625                 630                 635                 640

Lys

<210>  10
<211>  1032
<212>  PRT
<213>  Homo sapiens

<220>
<221>  SIGNAL

<222>  (1)..(33)
<223>

<220>
<221>  beta-propeller domain
<222>  (34)..(465)
<223>

<220>
<221>  Thigh domain
<222>  (466)..(618)
<223>

<220>
<221>  Genu
<222>  (619)..(626)
<223>

<220>
<221>  Calf-1 domain
<222>  (627)..(770)
<223>

<220>
<221>  Calf-2 domain
<222>  (771)..(977)
<223>

<220>
<221>  Transmembrane domain
<222>  (978)..(1000)
<223>

<220>
<221>  Cytoplasmic domain
<222>  (1001)..(1032)
<223>

<400>  10

<210>  11
<211>  185
<212>  PRT
<213>  Homo sapiens

<400>  11

Met Ala Trp Glu Ala Arg Arg Glu Pro Gly Pro Arg Arg Ala Ala Val
1               5                   10                  15

Arg Glu Thr Val Met Leu Leu Leu Cys Leu Gly Val Pro Thr Gly Arg
            20                  25                  30

Pro Tyr Asn Val Asp Thr Glu Ser Ala Leu Leu Tyr Gln Gly Pro His
        35                  40                  45

Asn Thr Leu Phe Gly Tyr Ser Val Val Leu His Ser His Gly Ala Asn
    50                  55                  60

Arg Trp Leu Leu Val Gly Ala Pro Thr Ala Asn Trp Leu Ala Asn Ala
65                  70                  75                  80

Ser Val Ile Asn Pro Gly Ala Ile Tyr Arg Cys Arg Ile Gly Lys Asn
                85                  90                  95

Pro Gly Gln Thr Cys Glu Gln Leu Gln Leu Gly Ser Pro Asn Gly Glu
            100                 105                 110

Pro Cys Gly Lys Thr Cys Leu Glu Glu Arg Asp Asn Gln Trp Leu Gly
        115                 120                 125

Val Thr Leu Ser Arg Gln Pro Gly Glu Asn Gly Ser Ile Val Thr Cys
    130                 135                 140

Gly His Arg Trp Lys Asn Ile Phe Tyr Ile Lys Asn Glu Asn Lys Leu
145                 150                 155                 160

Pro Thr Gly Gly Cys Tyr Gly Val Pro Pro Asp Leu Arg Thr Glu Leu
                165                 170                 175

Ser Lys Arg Ile Ala Pro Cys Tyr Gln
            180                 185

<210>  12
<211>  384
<212>  PRT
<213>  Homo sapiens

<400>  12

Met Ala Trp Glu Ala Arg Arg Glu Pro Gly Pro Arg Arg Ala Ala Val
1               5                   10                  15

Arg Glu Thr Val Met Leu Leu Leu Cys Leu Gly Val Pro Thr Gly Arg
            20                  25                  30

Pro Tyr Asn Val Asp Thr Glu Ser Ala Leu Leu Tyr Gln Gly Pro His
        35                  40                  45

Asn Thr Leu Phe Gly Tyr Ser Val Val Leu His Ser His Gly Ala Asn
    50                  55                  60

Arg Trp Leu Leu Val Gly Ala Pro Thr Ala Asn Trp Leu Ala Asn Ala
65                  70                  75                  80

Ser Val Ile Asn Pro Gly Ala Ile Tyr Arg Cys Arg Ile Gly Lys Asn
                85                  90                  95

Pro Gly Gln Thr Cys Glu Gln Leu Gln Leu Gly Ser Pro Asn Gly Glu
            100                 105                 110

Pro Cys Gly Lys Thr Cys Leu Glu Glu Arg Asp Asn Gln Trp Leu Gly
        115                 120                 125

Val Thr Leu Ser Arg Gln Pro Gly Glu Asn Gly Ser Ile Val Thr Cys
    130                 135                 140

Gly His Arg Trp Lys Asn Ile Phe Tyr Ile Lys Asn Glu Asn Lys Leu
145                 150                 155                 160

Pro Thr Gly Gly Cys Tyr Gly Val Pro Pro Asp Leu Arg Thr Glu Leu
                165                 170                 175

Ser Lys Arg Ile Ala Pro Cys Tyr Gln Asp Tyr Val Lys Lys Phe Gly
            180                 185                 190

Glu Asn Phe Ala Ser Cys Gln Ala Gly Ile Ser Ser Phe Tyr Thr Lys
        195                 200                 205

Asp Leu Ile Val Met Gly Ala Pro Gly Ser Ser Tyr Trp Thr Gly Ser
    210                 215                 220

Leu Phe Val Tyr Asn Ile Thr Thr Asn Lys Tyr Lys Ala Phe Leu Asp
225                 230                 235                 240

Lys Gln Asn Gln Val Lys Phe Gly Ser Tyr Leu Gly Tyr Ser Val Gly
                245                 250                 255

Ala Gly His Phe Arg Ser Gln His Thr Thr Glu Val Val Gly Gly Ala
            260                 265                 270

Pro Gln His Glu Gln Ile Gly Lys Ala Tyr Ile Phe Ser Ile Asp Glu
        275                 280                 285

Lys Glu Leu Asn Ile Leu His Glu Met Lys Gly Lys Lys Leu Gly Ser
    290                 295                 300

Tyr Phe Gly Ala Ser Val Cys Ala Val Asp Leu Asn Ala Asp Gly Phe
305                 310                 315                 320

Ser Asp Leu Leu Val Gly Ala Pro Met Gln Ser Thr Ile Arg Glu Glu
                325                 330                 335

Gly Arg Val Phe Val Tyr Ile Asn Ser Gly Ser Gly Ala Val Met Asn
            340                 345                 350

Ala Met Glu Thr Asn Leu Val Gly Ser Asp Lys Tyr Ala Ala Arg Phe
        355                 360                 365

Gly Glu Ser Ile Val Asn Leu Gly Asp Ile Asp Asn Asp Gly Phe Glu
    370                 375                 380

<210> 13
<211> 513
<212> PRT
<213> Homo sapiens

<400> 13

Met Ala Trp Glu Ala Arg Arg Glu Pro Gly Pro Arg Arg Ala Ala Val
1               5                   10                  15

Arg Glu Thr Val Met Leu Leu Leu Cys Leu Gly Val Pro Thr Gly Arg
            20                  25                  30

Pro Tyr Asn Val Asp Thr Glu Ser Ala Leu Leu Tyr Gln Gly Pro His
        35                  40                  45

Asn Thr Leu Phe Gly Tyr Ser Val Val Leu His Ser His Gly Ala Asn
    50                  55                  60

Arg Trp Leu Leu Val Gly Ala Pro Thr Ala Asn Trp Leu Ala Asn Ala
65                  70                  75                  80

Ser Val Ile Asn Pro Gly Ala Ile Tyr Arg Cys Arg Ile Gly Lys Asn
                85                  90                  95

Pro Gly Gln Thr Cys Glu Gln Leu Gln Leu Gly Ser Pro Asn Gly Glu
            100                 105                 110

Pro Cys Gly Lys Thr Cys Leu Glu Glu Arg Asp Asn Gln Trp Leu Gly
        115                 120                 125

Val Thr Leu Ser Arg Gln Pro Gly Glu Asn Gly Ser Ile Val Thr Cys
    130                 135                 140

Gly His Arg Trp Lys Asn Ile Phe Tyr Ile Lys Asn Glu Asn Lys Leu
145                 150                 155                 160

Pro Thr Gly Gly Cys Tyr Gly Val Pro Pro Asp Leu Arg Thr Glu Leu
                165                 170                 175

Ser Lys Arg Ile Ala Pro Cys Tyr Gln Asp Tyr Val Lys Lys Phe Gly
            180                 185                 190

Glu Asn Phe Ala Ser Cys Gln Ala Gly Ile Ser Ser Phe Tyr Thr Lys
        195                 200                 205

Asp Leu Ile Val Met Gly Ala Pro Gly Ser Ser Tyr Trp Thr Gly Ser
    210                 215                 220

Leu Phe Val Tyr Asn Ile Thr Thr Asn Lys Tyr Lys Ala Phe Leu Asp
225                 230                 235                 240

Lys Gln Asn Gln Val Lys Phe Gly Ser Tyr Leu Gly Tyr Ser Val Gly
                245                 250                 255

Ala Gly His Phe Arg Ser Gln His Thr Thr Glu Val Val Gly Gly Ala
            260                 265                 270

Pro Gln His Glu Gln Ile Gly Lys Ala Tyr Ile Phe Ser Ile Asp Glu
        275                 280                 285

Lys Glu Leu Asn Ile Leu His Glu Met Lys Gly Lys Lys Leu Gly Ser
    290                 295                 300

Tyr Phe Gly Ala Ser Val Cys Ala Val Asp Leu Asn Ala Asp Gly Phe
305                 310                 315                 320

Ser Asp Leu Leu Val Gly Ala Pro Met Gln Ser Thr Ile Arg Glu Glu
                325                 330                 335

Gly Arg Val Phe Val Tyr Ile Asn Ser Gly Ser Gly Ala Val Met Asn
            340                 345                 350

Ala Met Glu Thr Asn Leu Val Gly Ser Asp Lys Tyr Ala Ala Arg Phe
        355                 360                 365

Gly Glu Ser Ile Val Asn Leu Gly Asp Ile Asp Asn Asp Gly Phe Glu
    370                 375                 380

Asp Val Ala Ile Gly Ala Pro Gln Glu Asp Asp Leu Gln Gly Ala Ile
385                 390                 395                 400

Tyr Ile Tyr Asn Gly Arg Ala Asp Gly Ile Ser Ser Thr Phe Ser Gln
                405                 410                 415

Arg Ile Glu Gly Leu Gln Ile Ser Lys Ser Leu Ser Met Phe Gly Gln
        420                 425                 430

Ser Ile Ser Gly Gln Ile Asp Ala Asp Asn Asn Gly Tyr Val Asp Val
        435                 440                 445

Ala Val Gly Ala Phe Arg Ser Asp Ser Ala Val Leu Leu Arg Thr Arg
    450                 455                 460

Pro Val Val Ile Val Asp Ala Ser Leu Ser His Pro Glu Ser Val Asn
465                 470                 475                 480

Arg Thr Lys Phe Asp Cys Val Glu Asn Gly Trp Pro Ser Val Cys Ile
                485                 490                 495

Asp Leu Thr Leu Cys Phe Ser Tyr Lys Gly Lys Glu Val Pro Gly Tyr
        500                 505                 510

Ile

<210>  14
<211>  640
<212>  PRT
<213>  Homo sapiens

<400>  14

Met Ala Trp Glu Ala Arg Arg Glu Pro Gly Pro Arg Arg Ala Ala Val
1               5                   10                  15

Arg Glu Thr Val Met Leu Leu Leu Cys Leu Gly Val Pro Thr Gly Arg
            20                  25                  30

Pro Tyr Asn Val Asp Thr Glu Ser Ala Leu Leu Tyr Gln Gly Pro His
        35                  40                  45

Asn Thr Leu Phe Gly Tyr Ser Val Val Leu His Ser His Gly Ala Asn
    50                  55                  60

Arg Trp Leu Leu Val Gly Ala Pro Thr Ala Asn Trp Leu Ala Asn Ala
65                  70                  75                  80

Ser Val Ile Asn Pro Gly Ala Ile Tyr Arg Cys Arg Ile Gly Lys Asn
                85                  90                  95

Pro Gly Gln Thr Cys Glu Gln Leu Gln Leu Gly Ser Pro Asn Gly Glu
            100                 105                 110

Pro Cys Gly Lys Thr Cys Leu Glu Glu Arg Asp Asn Gln Trp Leu Gly
        115                 120                 125

Val Thr Leu Ser Arg Gln Pro Gly Glu Asn Gly Ser Ile Val Thr Cys
    130                 135                 140

Gly His Arg Trp Lys Asn Ile Phe Tyr Ile Lys Asn Glu Asn Lys Leu
145                 150                 155                 160

Pro Thr Gly Gly Cys Tyr Gly Val Pro Pro Asp Leu Arg Thr Glu Leu
                165                 170                 175

Ser Lys Arg Ile Ala Pro Cys Tyr Gln Asp Tyr Val Lys Lys Phe Gly
            180                 185                 190

Glu Asn Phe Ala Ser Cys Gln Ala Gly Ile Ser Ser Phe Tyr Thr Lys
        195                 200                 205

Asp Leu Ile Val Met Gly Ala Pro Gly Ser Ser Tyr Trp Thr Gly Ser
    210                 215                 220

Leu Phe Val Tyr Asn Ile Thr Thr Asn Lys Tyr Lys Ala Phe Leu Asp
225                 230                 235                 240

Lys Gln Asn Gln Val Lys Phe Gly Ser Tyr Leu Gly Tyr Ser Val Gly
                245                 250                 255

Ala Gly His Phe Arg Ser Gln His Thr Thr Glu Val Val Gly Gly Ala
            260                 265                 270

Pro Gln His Glu Gln Ile Gly Lys Ala Tyr Ile Phe Ser Ile Asp Glu
        275                 280                 285

Lys Glu Leu Asn Ile Leu His Glu Met Lys Gly Lys Lys Leu Gly Ser
    290                 295                 300

Tyr Phe Gly Ala Ser Val Cys Ala Val Asp Leu Asn Ala Asp Gly Phe
305                 310                 315                 320

Ser Asp Leu Leu Val Gly Ala Pro Met Gln Ser Thr Ile Arg Glu Glu
                325                 330                 335

Gly Arg Val Phe Val Tyr Ile Asn Ser Gly Ser Gly Ala Val Met Asn
            340                 345                 350

Ala Met Glu Thr Asn Leu Val Gly Ser Asp Lys Tyr Ala Ala Arg Phe

355                 360                 365

Gly Glu Ser Ile Val Asn Leu Gly Asp Ile Asp Asn Asp Gly Phe Glu
    370                 375                 380

Asp Val Ala Ile Gly Ala Pro Gln Glu Asp Asp Leu Gln Gly Ala Ile
385                 390                 395                 400

Tyr Ile Tyr Asn Gly Arg Ala Asp Gly Ile Ser Ser Thr Phe Ser Gln
                405                 410                 415

Arg Ile Glu Gly Leu Gln Ile Ser Lys Ser Leu Ser Met Phe Gly Gln
            420                 425                 430

Ser Ile Ser Gly Gln Ile Asp Ala Asp Asn Asn Gly Tyr Val Asp Val
        435                 440                 445

Ala Val Gly Ala Phe Arg Ser Asp Ser Ala Val Leu Leu Arg Thr Arg
    450                 455                 460

Pro Val Val Ile Val Asp Ala Ser Leu Ser His Pro Glu Ser Val Asn
465                 470                 475                 480

Arg Thr Lys Phe Asp Cys Val Glu Asn Gly Trp Pro Ser Val Cys Ile
                485                 490                 495

Asp Leu Thr Leu Cys Phe Ser Tyr Lys Gly Lys Glu Val Pro Gly Tyr
            500                 505                 510

Ile Val Leu Phe Tyr Asn Met Ser Leu Asp Val Asn Arg Lys Ala Glu
        515                 520                 525

Ser Pro Pro Arg Phe Tyr Phe Ser Ser Asn Gly Thr Ser Asp Val Ile
    530                 535                 540

Thr Gly Ser Ile Gln Val Ser Ser Arg Glu Ala Asn Cys Arg Thr His
545                 550                 555                 560

Gln Ala Phe Met Arg Lys Asp Val Arg Asp Ile Leu Thr Pro Ile Gln
                565                 570                 575

Ile Glu Ala Ala Tyr His Leu Gly Pro His Val Ile Ser Lys Arg Ser
            580                 585                 590

Thr Glu Glu Phe Pro Pro Leu Gln Pro Ile Leu Gln Gln Lys Lys Glu
        595                 600                 605

Lys Asp Ile Met Lys Lys Thr Ile Asn Phe Ala Arg Phe Cys Ala His
    610                 615                 620

Glu Asn Cys Ser Ala Asp Leu Gln Val Ser Ala Lys Ile Gly Phe Leu
625                 630                 635                 640

<210>  15
<211>  10
<212>  PRT
<213>  Homo sapiens

<400>  15

Gly Ser Ile Ser Lys Tyr Arg Ala Arg Thr
1               5                   10

<210>  16
<211>  2
<212>  PRT
<213>  Homo sapiens

<400>  16

Gly Asn
1

<210>  17
<211>  7
<212>  PRT
<213>  Homo sapiens

<400>  17

Gly Gly Tyr Ala Leu Gln Tyr
1               5

<210>  18
<211>  7
<212>  PRT
<213>  Homo sapiens

<400>  18

Asn Ser Leu Ser Phe Ser Ser
1               5

<210>  19
<211>  3
<212>  PRT
<213>  Homo sapiens

<400>  19

Cys Cys Leu
1

<210>  20
<211>  7
<212>  PRT
<213>  Artificial

<220>
<223>  amino acid sequence involved in cell adhesion

<400>  20

Ser Val Val Tyr Gly Leu Arg
1               5


<210>  21
<211>  13
<212>  PRT
<213>  Artificial

<220>
<223>  amino acid sequence involved in cell adhesion

<400>  21

Gln Asp His Ser Phe Ser Ile Val Ile Glu Thr Val Gln
1               5                   10


<210>  22
<211>  52
<212>  DNA
<213>  Artificial

<220>
<223>  QT primer for 3'-race

<400>  22
ccagtgagca gagtgacgag gactcgagct caagcttttt tttttttttt tt          52


<210>  23
<211>  18
<212>  DNA
<213>  Artificial

<220>
<223>  Q0 primer for 3'-race

<400>  23
ccagtgagca gagtgacg                                                18


<210>  24
<211>  30
<212>  DNA
<213>  Artificial

<220>
<223>  primer for human alpha 4 integrin

<400>  24
tgtctcgagt ggccgtttag tgttgaatgt                                   30

<210>  25
<211>  21
<212>  DNA
<213>  Artificial

<220>
<223>  primer for human alpha 4 integrin

<400>  25
actggtggtt gctatggagt a                                                21


<210>  26
<211>  21
<212>  DNA
<213>  Artificial

<220>
<223>  primer for human alpha 4 integrin

<400>  26
cttttcggtc tgattctgct g                                                21


<210>  27
<211>  18
<212>  DNA
<213>  Artificial

<220>
<223>  primer

<400>  27
gaggactcga gctcaagc                                                    18


<210>  28
<211>  21
<212>  DNA
<213>  Artificial

<220>
<223>  primer

<400>  28
cacttcagcc aattcttcag c                                                21


<210>  29
<211>  58
<212>  DNA
<213>  Artificial

<220>
<223>  primer for human alpha 4 integrin mutant 1-1-2

<400>  29
ttactctaga ctatttatcg tcatcatctt tgtagtcatt accttcaaag ccatcatt        58


<210>  30
<211>  58

<212>  DNA
<213>  Artificial

<220>
<223>  primer for human alpha 4 integrin mutant 1-2-1

<400>  30
tcgttctaga ctatttatcg tcatcatctt tgtagtctgt cctagctctg tacttgct       58


<210>  31
<211>  58
<212>  DNA
<213>  Artificial

<220>
<223>  primer for human alpha 4 integrin mutant 7-3-2

<400>  31
ccactctaga ctatttatcg tcatcatctt tgtagtcata ttgtagggca tacccacc       58


<210>  32
<211>  58
<212>  DNA
<213>  Artificial

<220>
<223>  primer for human alpha 4 integrin mutant 10-6-3

<400>  32
ttgatctaga ctatttatcg tcatcatctt tgtagtctga ggaaaagctg agagagtt       58


<210>  33
<211>  58
<212>  DNA
<213>  Artificial

<220>
<223>  primer for human alpha 4 integrin mutant 10-7-2

<400>  33
cccatctaga ctatttatcg tcatcatctt tgtagtcgag acaacacttc aaaaaccc       58


<210>  34
<211>  58
<212>  DNA
<213>  Artificial

<220>
<223>  primer for human alpha 4 integrin mutant 9-5-4

<400>  34
cgtttctaga ctatttatcg tcatcatctt tgtagtcctt caaaaaccca atctttgc       58


<210>  35
<211>  29
<212>  DNA
<213>  Artificial

<220>
<223>  5' primer for N-terminal signal sequence lacked alpha 4 integrin
       mutant 1-2-1

<400>  35
gaggaattct acaacgtgga cactgagag                                        29


<210>  36
<211>  32
<212>  DNA
<213>  Artificial

<220>
<223>  3' primer for N-terminal signal sequence lacked alpha 4 integrin
       mutant 1-2-1

<400>  36
cgtctcgagt tatgtcctag ctctgtactt gc                                    32


<210>  37
<211>  30
<212>  DNA
<213>  Artificial

<220>
<223>  primer for PCR

<400>  37
cgaggatcct gaatgttctc caccaagagc                                       30


<210>  38
<211>  30
<212>  DNA
<213>  Artificial

<220>
<223>  primer for PCR

<400>  38
ttactcgaga cgggtcttct gaacaggatt                                       30


<210>  39
<211>  29
<212>  DNA
<213>  Artificial

<220>
<223>  primer for PCR

<400>  39
gctaagcttc tctcgactgt agcccatcg                                        29


<210>  40
<211>  29
<212>  DNA
<213>  Artificial

<220>

<223>  primer for PCR

<400>  40
ccatctagag caactgctga gaggaaacc                                        29


<210>  41
<211>  57
<212>  DNA
<213>  Artificial

<220>
<223>  primer for PCR

<400>  41
ctgtctagat tacttgtcat cgtcatcctt gtagtcctgg tttttctgga cccagtc        57


<210>  42
<211>  5
<212>  PRT
<213>  Artificial

<220>
<223>  amino acid sequence involved in cell adhesion

<400>  42

Gly Arg Gly Asp Ser
1               5


<210>  43
<211>  13
<212>  PRT
<213>  Artificial

<220>
<223>  peptide used to develop EAE model mouse

<400>  43

His Ser Leu Gly Lys Trp Leu Gly His Pro Asp Lys Phe
1               5                   10